(19) Europäisches Patentamt
European Patent Office
Office européen des brevets

(11) Publication number: **0 098 041**
**B1**

(12) # EUROPEAN PATENT SPECIFICATION

(45) Date of publication of patent specification: **02.08.89**

(21) Application number: **83302790.7**

(22) Date of filing: **17.05.83**

(51) Int. Cl.$^4$: **C 07 D 493/04,** C 07 D 493/14, C 07 D 519/00, C 07 D 307/93, A 61 K 31/365 // (C07D493/04, 307:00, 303:00),(C07D493/14, 307:00, 303:00, 303:00),(C07D519/00, 493:00, 493:00)

(54) **Sesquiterpene lactones, extracts containing them, their preparation and pharmaceutical use.**

(30) Priority: **19.05.82 GB 8214572**
**22.09.82 GB 8227062**

(43) Date of publication of application:
**11.01.84 Bulletin 84/02**

(45) Publication of the grant of the patent:
**02.08.89 Bulletin 89/31**

(84) Designated Contracting States:
**AT BE CH DE FR IT LI LU NL SE**

(56) References cited:
**JOURNAL OF ORGANIC CHEMISTRY, vol. 44, no. 22, 1979, Columbus, OH (US); F.S.EL-FERALY et al.: "Isolation and characterization of peroxycostunolide (verlotorin) and peroxyparthenolide from magnolia grandiflora. Carbon-13 nuclear magnetic resonance spectroscopy of custunolide and related compounds", pp. 3952-3955**

(73) Proprietor: **R.P. SCHERER CORPORATION**
**2075 West Big Beaver Road**
**Troy Michigan 48099 (US)**

(72) Inventor: **Johnson, Edward Stewart**
**The Boyne Boyndon Road**
**Maidenhead Berkshire SL6 4EU (GB)**
Inventor: **Hylands, Peter John**
**1, The Cottages Stubbings Estate**
**Maidenhead Thicket Berkshire SL6 6QL (GB)**
Inventor: **Hylands née Jessup, Deborah Margaret**
**1, The Cottages Stubbings Estate**
**Maidenhead Thicket Berkshire SL6 6QL (GB)**

(74) Representative: **Paget, Hugh Charles Edward et al**
**MEWBURN ELLIS & CO. 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 098 041 B1**

(56) References cited:

**Chemical Abstracts vol. 98, no. 25, 20 June 1983, Columbus, OH (US); F.BOHLMANN et al.: "Naturally occurring terpene derivatives. Part 454. Sesquiterpene lactones and other constituents from tanacetum parthenium", p. 319, no. 212794k**

**Dictionary of organic compounds, 5th ed., Chapman & Hall (1982), pp. 1233,2738-2740, 2818, 2820**

**TETRAHEDRON LETTERS, vol. 1977, no. 23, Oxford (GB); F.S.EL-FERALY et al.: "Peroxycustunolide and peroxyparthenolide: The cytotoxic germacranolide hydroperoxides from magnolia grandiflora. Structural revision of verlotorin and artemorin", pp. 1973-19**

**Description**

This invention relates to sesquiterpene lactones and sesquiterpene lactone-containing plant extracts and preparations for pharmaceutical use, and particularly to extracts and preparations which are useful in the treatment of migraine and various arthritic and bronchial conditions.

The incidence of migraine is said to be in the region of 12% of the population. Its precise causes have not been determined but certain elements in its causation are well documented.

Neurohumoral and local hormone (autacoid) factors such as noradrenaline, 5-hydroxytryptamine (serotonin), histamine and prostaglandins are implicated in migraine probably acting at the cerebrovascular level.

There is much evidence to suggest that a disturbance of blood vessel diameter is involved in the migraine process. Thus reports have demonstrated a constriction of cerebral cortical vessels followed by a dilatation during a migraine attack. The vasoconstriction is believed to be due to the release of amines such as noradrenaline from neurones and 5-hydroxytryptamine from platelets. Histamine is also released from mast cells and plays an important role in the migraine variant known as cluster headache (migrainous neuralgia). Drugs with antinoradrenaline, anti 5-hydroxytryptamine and antihistamine activities are all used to alleviate the symptoms of migraine. Evidence that prostaglandins (PG), such as $PGE_2$, $PGF_{2\alpha}$, $PGI_2$ (prostacyclin) or thromboxane $A_2$ are involved comes from the fact that non-steroidal anti-inflammatory drugs such as aspirin, which act by inhibiting the synthesis of prostaglandins, are highly effective for both the prophylactic and acute treatment of migraine.

As to the causes of arthritic conditions, the suppression of prostaglandin synthesis by non-steroidal anti-inflammatory drugs such as aspirin or indomethacin strongly implicates prostaglandins as inflammatory mediators at least in rheumatoid arthritis. Prostaglandin-like material is present in inflammatory perfusates of experimental animals. The E-prostaglandins in low concentrations are striking potentiators of the pain-producing properties of other agonists such as histamine, 5-hydroxytryptamine and plasmakinins (e.g. bradykinin) as well as other features of the inflammatory response such as increased vascular permeability, erythema, white cell accumulation and platelet aggregation.

Patients with bronchial asthma and chronic bronchitis suffer attacks of wheezing and difficulty in breathing. These are due to bronchial narrowing from spasm of the bronchial smooth muscle or from sticky secretions, or more commonly from a combination of the two. The precise causes of the bronchoconstriction in asthma have not been fully elucidated, but it is believed endogenous neurohumoral and autacoid spasmogens such as acetylchlorine, 5-hydroxytryptamine (serotonin), histamine, some prostaglandins and leukotrienes may be involved. Allergic reactions to substances in inhaled dust or those absorbed from food may be manifested by bronchoconstriction or urticaria ("nettle-rash") or circulatory collapse caused by the release of histamine or other endogenous substances.

Drugs with spasmolytic actions such as aminophylline are used to treat bronchospasm. Drugs with antihistamine activity are also used in asthma and for wheezing and other symptoms associated with allergic phenomena such as hay fever.

It has been known for many years that sesquiterpene lactones and present in plants, for example, in the family Compositae (see Yoshio, Mabry and Timmermann, "Sesquiterpene Lactones, Chemistry, NMR and Plant Distribution", University of Tokyo Press 1973).

Of these plants *Tanacetum parthenium* (formerly called *Chrysanthemum parthenium* and commonly known as feverfew, featherfoil, flirtwort and Bachelor's Buttons) has been put forward in herbal medicine as a possibility effective treatment for migraine when consumed. No serious medical study of this treatment for migraine has apparently been published, nor has there been any consideration of what ingredient or ingredients of the plant might be involved in this alleged efficacy.

Many years ago, it was reported that the sesquiterpene lactones parthenolide and santamarine may be extracted from feverfew using light petroleum and chloroform respectively (see Soucek, Heront and Sorm, Coll. Czech. Chem, Comm., (1961), *26*, 803 and Romo et al, Tetrahedron, (1965), *21*, 1741 respectively). It was also reported that the sesquiterpene lactones chrysartemin A and B are present in Tanacetum parthenium (see Dictionary of Organic Compounds, 5th Edn., Vol 1, A-Clor, page 1233). In those publications, there was no indication of any possible medical use for the sesquiterpene lactones.

Some cytotoxic activity of parthenolide and several other sesquiterpene lactones has also been reported some time ago (Lee et al, Cancer Research, (1971), *31*, 1649). There is, however, no suggestion in this article that any sesquiterpene lactone or extract containing it may be useful in the treatment of migraine, arthritis or bronchial complaints.

More recently it has been shown that certain sesquiterpene lactones are potent inhibitors of carrageenan-induced edma and chronic adjuvant-induced arthritis in rodents (see Hall, Starnes, Lee and Waddell, J. Pharm. Sci., (1979), *68*, 537). This article does not, however, suggest that any sesquiterpene lactone has pharmaceutical activity against any complaint, let alone against migraine.

By repeated selective extraction of feverfew material, and extensive *in vitro* testing of the extracts for biological activity, in particular spasmoytic activity in smooth muscle tests, we have found surprisingly that, of the very many constituents of the plant, certain of the compounds within the range of sesquiterpene lactones present have properties strongly indicative of efficacy in the treatment of migraine. This is particularly surprising because the existence of some such compounds in the plant has been known for

many years and yet there has been no disclosure or suggestion anywhere in the literature that they possess any useful activity against migraine. Furthermore, at least some of the reports on the use of feverfew in the treatment of migraine suggest taking an aqueous extract of the plant as a "tea". This points away from sesquiterpene lactones being active ingredients since they are unlikely to be extracted from the plant by this method.

The indications are that such compounds are also likely to be efficacious in the treatment not only of migraine but also of bronchial asthma and arthritic conditions such as rheumatoid arthritis, osteoarthritis and related arthritides. This could not have been predicted from the completely unrelated cytotoxic activity reported by Lee et al (see above).

According to one aspect of the invention, a sesquiterpene lactone is used for the treatment of migraine, asthmatic, bronchial or arthritic conditions, which sesquiterpene lactone is derived from the plant *Tanacetum parthenium* (hereinafter called "feverfew") by extraction with an organic solvent which is essentially non-polar, for example, light petroleum, hexane, chloroform or an oil, for example, a saturated or unsaturated long chain (e.g. $C_{10-25}$) hydrocarbon or fatty acid, e.g. a commercial available vegetable or animal oil such as coconut oil, soya bean oil, a fish oil, there derivatives such as polyoxyethylated fatty acids, reconstituted glycerides and esters, e.g. sorbitan esters of the above compounds.

The sesquiterpene-lactone may be present in an extract of the plant, which extract may be obtained by mixing dried, pulverized feverfew leaves with the non-polar solvent and leaving the mixture to stand to allow the extraction to take place. Alternatively, the dry powder may be exhaustively extracted with the non-polar organic solvent, for example in a Soxhlet apparatus. Classes of sesquiterpene lactones which include compounds which we find likely to be effective against one or more or migraine, asthma and arthritic conditions are germacranolides and guaianolides, including pseudoguaianolides, especially those sesquiterpene lactones having an α-methylene substituent in the lactone ring. The extract may contain a plurality of sesquiterpene lactones such as these.

A germacranolide which can be extracted from feverfew is parthenolide of the formula

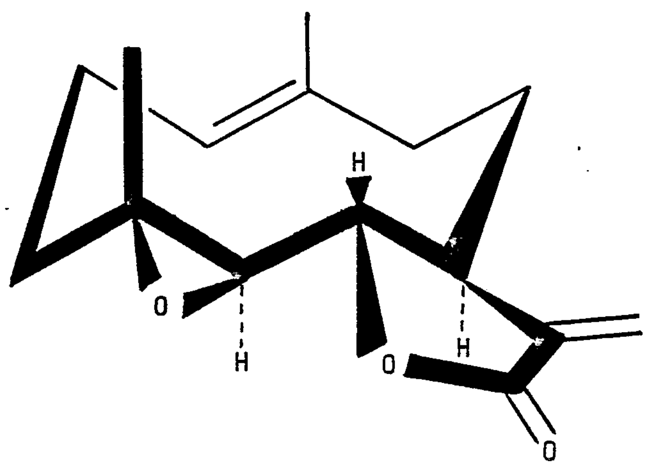

and we find that this compound and extracts from feverfew which contain it are particularly likely to be effective against migraine, asthma and various arthritic conditions.

The invention additionally provides a germacranolide which contains at least two germacrane nuclei in its molecule.

Because such germacranolides e.g. dimers and trimers, have more than one germacrane nucleus, each of which preferably carries out or more functional substituents, thay are, we believe, likely to be more effective than germacranolides containing a single germacrane nucleus. Hence we predict that they can be administered at lower dosage levels to achieve a comparative effect.

A particular such germacranolide and feverfew extracts containing it which we find likely to be effective has the formula

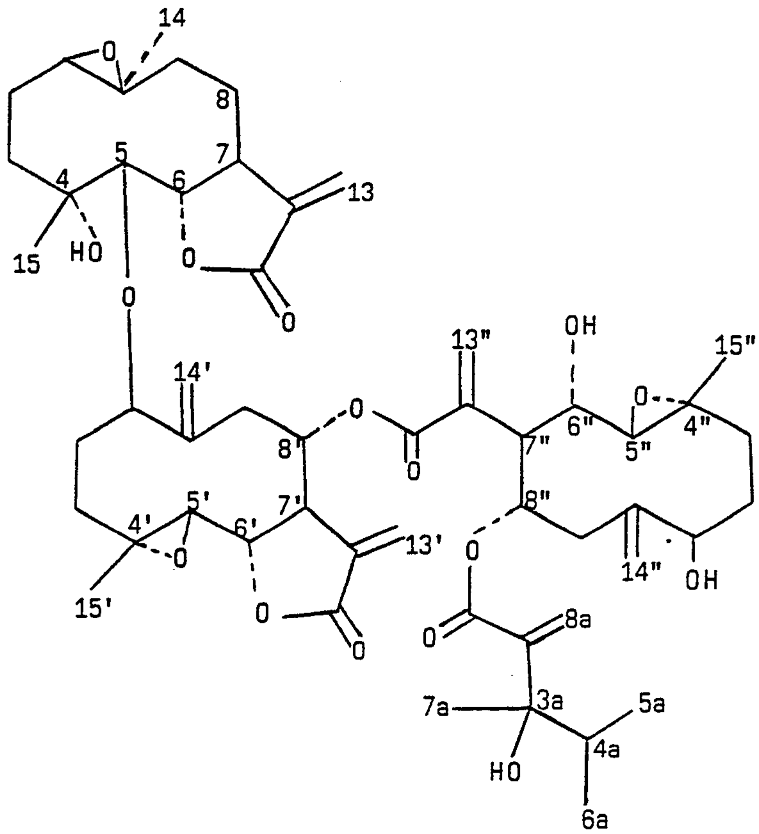

(5)

We have obtained this compound by extraction from feverfew with a non-polar solvent and believe it to be newly isolated. We call it "chrysanthemonin". The compound appears to be nowhere described in the literature.

We have also hydrolysed chrysanthemonin to yield a compound of the formula

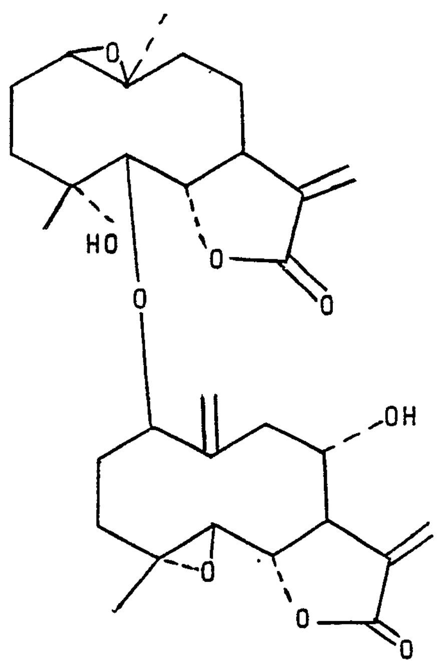

(6)

Particular guaianolides and feverfew extracts containing them which we find are likely to be effective have the respective formulae

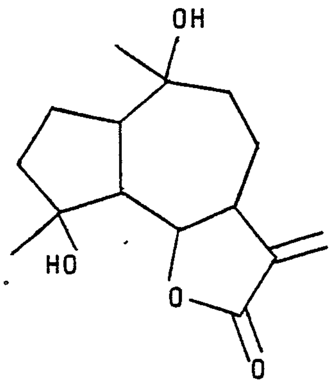

(3)'

and

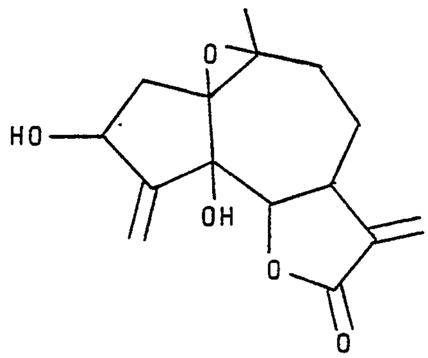

(4)

We have obtained these compounds by extraction from feverfew with a non-polar solvent and believe them to be newly isolated. We call them "partholide" (3)' and "chrysanthemolide" (4). The compounds appear to be nowhere described in the literature.

Such guaianolides have an oxygen functionality at the 10-position of the guaiane nucleus provided, for example, by an epoxy linked to a carbon atom adjacent to the said 10-position, or a hydroxyl, ether or ester group, or pharmaceutically acceptable derivatives of such compounds.

Guaianolides having multiple functionality, such as those having oxygen functionality at the 10-position, are, we believe likely to be more effective than guaianolides having no such multiple functionality.

A sesquiterpene lactone-containing extract or preparation for use in accordance with the invention may be obtained from feverfew in the following manner.

Leaves of feverfew are freeze-dried and then powdered. The dry powder is exhaustively extracted in a Soxhlet apparatus with an organic solvent for example, petroleum ether (i.e. light petroleum) boiling range 60/40°. The extract is evaporated to dryness and chromatographed, e.g. on a silica gel column. Elution of the column is effected by increasing polarity of solvent, e.g. from 100% petroleum ether boiling range 60/40° to 40% methanol in chloroform.

Fractions are collected and combined as appropriate having regard to their thin layer chromatography. The combined fractions are tested for spasmolytic activity as later described. Those fractions showing 100% inhibition of the agonists are further sub-fractionated. These sub-fractions are then tested. Optionally, the active constituents may be isolated after further purification (usually preparative thin layer chromatography). The pure compounds can be re-crystallised from appropriate solvents and analysed by spectroscopic techniques, for example, $^{1}H$ NMR, $^{13}C$ NMR, mass spectroscopy, infrared spectroscopy or ultraviolet spectroscopy.

They may then be made up into compositions in accordance with the invention for pharmaceutical use by conventional procedures.

Alternatively a sesquiterpene lactone-containing extract for use in accordance with the present invention may be obtained from feverfew by mixing the dried, pulverized leaves of the plant with a non-polar solvent to form a suspension and allowing the mixture to stand to allow extraction of sesquiterpene lactones from the plant.

The suspension may then be stabilized by use of suitable solid fat mixtures, with or without the presence of beeswax or other mineral waxs, colloidal silica, derivatives of cellulose such as carboxy methylcellulose and/or natural gums such as agar or Xanthan, starches, starch derivatives and other suitable carbohydrates, with or without the addition of wetting agents such as sorbitan fatty esters and lecithin.

Where the extract containing the sesquiterpene lactone is obtained by forming a stable suspension of dried, pulverized feverfew leaf in a non-polar solvent, a preparation in accordance with the invention may

be formed by encapsulating the suspension in either a hermetically sealed one-piece soft shell or a two-piece hard shell gelatin capsule representing a one unit dose, preferably an approximate amount of 0.25 to 200 mg, of feverfew leaf with or without the other pharmaceutically active substances.

Alternatively the dried, pulverized feverfew may be allowed to stand in the non-polar solvent for sufficient time to allow extraction, e.g. 14 days, and then strained to form a macerate of filtered to leave an extract solution.

The suspension macerate or extract solution may be mixed with surfactive agents or mixtures thereof, such as polyethoxy fatty acid sorbitan esters, mono and di glycerides, acetylated glycerides, to form a self-emulsifying system.

A sesquiterpene lactone containing extract for use in accordance with the invention as a medicament may be administered as it is, i.e. in the form of a plant extract or purified sesquiterpene lactone alone, but is usually admixed with a pharmaceutically acceptable excipient. The medicament may comprise one or more extracts and/or synthesized sesquiterpene lactones.

For the treatment of migraine the medicaments may be administered orally or parenterally and conveniently take the form of a tablet or liquid suspension. They may additionally include, in addition to the sesquiterpene lactones, other known anti-migraine preparations, analgesics and antiemetics as

Propranolol Hydrochloride,
Ergotamine Tartrate,
Methylsergide Maleate,
Dihydroergotamine Mesylate,
Clonidine Hydrochloride,
Ismetheptene Mucate,
Buclizine Dihydrochloride,
Metoclopramide Hydrochloride,
Pizotifen Hydrogen Malate,
aspirin and other non-steroidal anti-flammatory agents,
paracetamol and other minor analgesics,
pentazocine hydrochloride,
prochlorperazine,
Caffeine,
Meprobamate,
Ethoheptazine Citrate,
Zomepirac,
Meptazinol Hydrochloride,

The dosage of active ingredient may be from 0.25 to 200 milligrams per day. In general, from 0.25 to 20 mg per day is sufficient but, in the treatment of an acute attack of migraine or allied disorder the optimal dosage range will be higher at about 2 milligrams to 200 milligrams per day.

It is best given in an oral form made up as a tablet, capsule, or as a liquid suspension on a daily basis or regular basis as prescribed by a doctor. One dose should be effective but can be repeated as necessary. However, circumstance may arise where it is best administered by suppository, inhalation or injection.

For oral administration, the medicament may contain any conventional tabletting or capsuling carrier, or as a suspension in any orally acceptable non-toxic liquid carrier. If desired, the drug may be provided in encapsulated form for sustained release over a period of time. For parenteral administration the drug may be provided as a suspension in any suitable, sterile injection medium, e.g. sterile aqueous saline solution. Given the desired dosage rates indicated above, the appropriate method of formulation of the drug in a form suitable for oral or parenteral administration will be obvious to persons skilled in the art. The same applies for rectal administration, which is also feasible.

Excellent efficacy of a medicament in accordance with this invention is thought likely (a) in the treatment of classical and common migraine; (b) in the treatment of migrainous neuralgia (cluster headache); and (c) in the treatment of premenstrual and menstrual migraine and other headaches.

The medicaments are likely to be useful in the prevention of the above types of headache, being effective in reducing the frequency, severity and duration of the attacks. They should also be effective in reducing the nausea and vomiting associated with such attacks, or nausea and vomiting as isolated symptoms. They can probably also be used to treat the acute attacks of the above sorts of headache reducing their duration, severity and associated symptoms.

The precise reason for the effectiveness of these medicaments in the treatment of migraine is not known but it is hypothesised that in the disorder of migraine and its associated conditions there is an altered reactivity of the cerebral blood vessels to biogenic amines and prostaglandins released locally or into the systemic circulation. Based on this hypothesis, it is believed that the effects of a medicament in accordance with the invention in the treatment of migraine may be the result of a stabilisation of the blood vessel smooth muscle cell membranes leading to a direct inhibition or reduction of their responsiveness to biogenic amines and prostaglandins or to a indirect action by the inhibition of the effects of these substances on the nerves to the intracranial blood vessels.

We find that medicaments in accordance with the invention block the actions of neurohumoral transmitters and autacoids such as acetylcholoine, noradrenaline, 5-hydroxytryptamine, histamine, bradykinin and prostaglandin $E_2$ on smooth muscle. Furthermore chronic administration of the extracts to guinea-pigs causes a progressive decrease in the reactivity of their smooth muscle to 5-hydroxytryptamine and histamine. Indeed we find that such tests may be carried out on a particular medicament to determine its likely efficacy in the treatment of migraine.

For the treatment of arthritic conditions, medicaments in accordance with the invention may be administered orally, rectally, parenterally or by inhalation and conveniently take the form of a tablet, capsule, suppository or liquid suspension.

The dosage of active ingredient may be from 0.25 to 200 mg per day. In general, from 0.25 to 20 mg per day is sufficient but, in the treatment of an acute attack of rheumatoid arthritis or allied disorder the optimal dose range will be higher at about 2 milligrams to 200 milligrams per day.

It is best given in an oral form made up as a tablet or as a liquid suspension on a daily basis or regular basis as prescribed by a doctor. One dose should be effective but can be repeated as necessary. However circumstances may arise where it is best administered by suppository, inhalation or injection.

A medicament in accordance with the invention may additionally include other antiarthritis agents including non-steroidal antiinflammatory drugs, analgesics, skeletal muscle relaxants, steroids, gold and penicillanine.

Examples of such agents are

aspirin,
indomethacin,
piroxicam,
benorylate,
ibuprofen,
paracetamol,
salicylamine,
diflunisal,
ethoheptazine,
fenoprofen (calcium fenoprofate),
flufenamic acid,
mefenamic acid,
naproxen sodium,
ketoprofen,
phenylbutazone,
sulindac,
penicillamine,
salsalate,
fenclofenac,
flurbiprofen,
fenbufen,
feprazone,
sodium aurothiomalate,
naproxen,
benoxaprofen,
aloxiprin,
hydroxychloroquine sulphate,
azapropazone,
oxyphenbutazone,
tolmetin,
choline magnesium trisalicylate,
diclofenac,
adrenal steroids such as prednisone or prednisolone.

As to oral administration of the medicament for arthritis treatment, see the remarks above.

Efficacy of a medicament in accordance with this invention is indicated (a) in the treatment of rheumatoid arthritis; (b) in the treatment of osteoarthritis; and (c) in the treatment of arthritis associated with Felty's syndrome, Still's disease, systemic lupus erythematosus, polyarteritis nodosa, scleroderma, gout, achalasia of the cardia, Crohn's disease, chronic brucellosis, ankylosing spondylitis, sarcoidosis, psoriasis and gonorrhoea.

The medicaments are likely to be useful in the prevention of the above arthritides, being effective in reducing the frequency, severity and duration of the attacks. The medicaments can also be used to treat the acute attacks of the above arthritides reducing their duration, severity and associated symptoms.

The precise reason for the likely effectiveness of these medicaments in the treatment of arthritis is not known but it is hypothesised that in rheumatoid arthritis and its associated conditions prostaglandins and

other substances such as histamine, 5-hydroxytryptamine and bradykinin are released in and around joints. Prostaglandins potentiate the pain-inducing properties of the other substances. Based on this hypothesis, it is believed that the likely effects of a medicament in accordance with the invention in the treatment of arthritic conditions may be the result of their antiprostaglandin, antihistamine, anti-5-hydroxytryptamine and antibradykinin actions.

We find that medicaments in accordance with the invention have marked spasmolytic actions against a wide variety of smooth muscle spasmogens. Thus they block the actions of histamine, 5-hydroxytryptamine, bradykinin and prostaglandin $E_2$. Thus they oppose the pharmacological actions of the most commonly-implicated mediators of chronic inflammation. We find that such tests may be carried out on a particular medicament to determine its likely efficacy in the treatment of rheumatoid arthritis and associated conditions.

For the treatment of asthma, medicaments in accordance with the invention may be administered orally, rectally, parenterally or by inhalation.

The dosage of active ingredient may be from 0.25 to 200 milligrams per day. In general, from 0.25 to 20 mg per day is sufficient, but in the treatment of an acute attack of asthma or allied disorder the optimal dose range will be higher at about 2—200 mg per day.

It is best given in an oral form made up as a tablet or capsule or as a liquid suspension on a daily or regular basis as prescribed by a doctor. One dose may be effective but can be repeated as necessary. However, circumstances may arise where it is best administered by suppository, inhalation or injection.

In addition to the sesquiterpene lactone, the medicament may also contain other ingredients such as bronchodilator, antihistamine and anti-infective agents, examples of which agents are

isoprenaline sulphate,
orciprenaline,
adrenaline,
terbutaline sulphate,
theophylline,
choline theophyllinate,
aminophylline,
ephedrine hydrochloride,
papaverine hydrochloride,
ipratropium bromide,
atropine methonitrate,
beclomethasone dipropionate,
fenoterol hydrobromide,
betamethasone,
isoetharine mesylate or hydrochloride,
phenylephrine hydrochloride or bitartrate,
thenyldiamine hydrochloride,
reproterol hydrochloride,
deptropine citrate,
butethamate citrate,
acepifylline,
diphenylpyraline hydrochloride,
sodium cromoglycate,
etamiphylline camsylate,
theophylline monoethanolamine,
etafedrine hydrochloride,
bufylline,
guaiphenesin,
diphenyldramine hydrochloride and other histamine $H_1$-receptor antagonists,
diprophylline,
methoxyphenamine hydrochloride,
rimiterol hydrobromide,
hyoscine hydrobromide,
salbutamol sulphate,
ketotifen hydrogen fumarate,
pseudo-ephedrine hydrochloride,
bromhexine hydrochloride, and
antifungal, antibacterial and antiviral agents.

As to oral administration for treatment of asthma, see the remarks above.

Efficacy of a medicament in accordance with this invention is indicated (a) in the treatment of bronchial asthma; (b) in the treatment of bronchoconstriction associated with chronic bronchitis; (c) in the treatment of symptoms associated with histamine release in allergic hypersensitivity phenomena such as hay fever and anaphylaxis.

The medicaments can also be co-administered with other bronchodilator, antihistamine and anti-infective agents as enumerated above.

The precise reason for the proposed effectiveness of these substances in the treatment of asthma and other allergic reactions, rests on their ability to prevent or inhibit smooth muscle spasm caused by a variety of endogenous substances such as acetylcholine, histamine, 5-hydroxytryptamine, bradykinin and prostaglandins capable of being released locally or into the systemic circulation. Thus the excellent effects of a medicament in accordance with the invention in the treatment of bronchoconstriction may be the result of a stabilisation of the smooth muscle cell membranes leading to a direct inhibition or reduction of their responsiveness to biogenic amines, prostaglandins and polypeptides, or to an indirect action by the inhibition of the effects of the active ingredients on the nerves to the bronchial musculature and/or mucus secreting cells.

We find that medicaments in accordance with the invention block the spasms (contractions) of smooth muscle caused by acetylcholine, 5-hydroxytryptamine, histamine, prostaglandin $E_2$ and bradykinin. Indeed we find that such spasmolytic activity tests can be carried out on a particular medicament to deterine its likely efficacy in the treatment of bronchial complaints.

The extraction from feverfew of sesquiterpene lactones for use in the manufacture of spasmolytically active medicaments and compositions in accordance with the invention and the efficacy of such medicaments and compositions will now be illustrated in more detail with reference to the accompanying drawings and the following Examples

The accompanying drawing illustrates the extraction and isolation procedure starting from a light petroleum extract of feverfew.

In the drawing, "PTLC" means "Preparative Thin Layer Chromatography", the numbers represent combined fractions, i.e. 1—8 and new compounds or mixtures are indicated by the following codes:—

DJ 156a — partholide of formula (3) above
DJ 177b — chrysanthemolide of formula (4) above
DJ 140a chrysanthemonin of formula (5) above
DJ 140a H2 — compound of formula (6) above
DJ 61a — a long chain fatty acid ester
DJ 192a — a mixture of parthenolide and an unidentified terpene.
DJ 124a — a mixture of fatty acid esters
DJ 179al — possibly a mixture of reynosin, chrysartemin A and a new sesquiterpene lactone dihydroreynosin.
DJ 179c — a complex sesquiterpene lactone containing an α-methylene-γ-lactone.

## GENERAL DETAILS

A chromatography

(a) *Adsorbents*

Silica gel was the adsorbent used for all chromatography. The silica gel used for column chromatography was Kiosolgel 60 (Merck) and for thin-layer chromatography (TLC) Kieselgel G (Merck) and Kieselgel $GE_{254}$ (Merck). TLC plates for routine work, both analytical and preparative, were prepared by mixing 15 g Kieselgel G and 15 g Kieselgel $GF_{254}$ with 60 ml distilled water Kieselgel is a Trade Mark. The slurry was spread in a layer 0.25 mm thick on 20 × 20 cm glass plates. The plates were dried in the open air and then activated at 105°C for one hour. Silver nitrate impregnated plates where prepared by mixing 30 g Kieselgel G with 60 ml of a 10% w/v solution of silver nitrate in distilled water. The slurry was spread in a layer as before.

(b) *Solvent systems*

The solvents used for elution of columns and the development of TLC plates are detailed in the appropriate sections.

(c) Detection of components on TLC plates

Short wave ultraviolet light (254 nm) and spraying with 20% v/v sulphuric acid in distilled water followed by heating at 105°C for 10 minutes were used for the detection of components.

B Infrared Spectra

Obtained is solid films, Nujol mulls, chloroform solutions or KBr discs using a Unicam SP 200 spectrometer. Nujol and Unicam are Trade Marks.

C Ultraviolet Spectra

Taken in spectroscopic ethanol at a pathlength of 1 cm using a Porkin-Elmer (Trade Mark) Lambda 3 UV/VIS spectrophotometer.

D Melting Point

Taken with an Electrothermal melting point apparatus and are uncorrected.

E Hydrogen-1 nulcear magnetic resonance spectra

Recorded at 400 MHz using a Bruker (Trade Mark) WH 400 spectrometer or at 200 MHz on a Nicolet (Trade Mark) NT 200 spectrometer in deuterochloroform using tetramethylsilane (TMS) as internal standard.

F Carbon-13 Nuclear Magnetic Resonance Spectra

Recorded at 100.6 MHz using a Bruker WH 400 spectrometer in deuterochloroform using tetramethylsilane as internal standard.

Both $^1H$ and $^{13}C$ resonances are given in δ (TMS δ 0.0) and the following abbreviations have been used: *s*, singlet; *d*, doublet; *t*, triplet; *q*, quartet; *m*, multiplet and J, coupling constant.

G Mass Spectra

Recorded on an AEI MS 902 high resolution mass spectrometer or a VG Micromass (Trade Mark) 16S spectrometer at 18 or 70 eV with direct inlet temperatures of between 180°C and 240°C.

2. Extraction of tanacetum parthenium with light petroleum (b.r. 40—60°C)

5.8 kg of the leaves of *Tanacetum parathonium* were collected in January 1980 from the Chelsea Physic Garden, Royal Hospital Road, London SW3 and freeze-dried using a Chemical Laboratory Instruments SB4 freeze-drier. A voucher specimen of the dried aerial parts of the plant was placed in the museum at Chelsea College. The freeze-dried leaves lost 82.8% on drying to give a final weight of 1 kg. These were powdered and exhaustively extracted in a Soxhlet apparatus with light petroleum b.r. 40—60°C for 7 days. The extract was evaporated to dryness using a rotary evaporator under reduced pressure to give a yellow oily residue weighing 44 g.

3. Spasmolytic activity of the light petroleum extract

50 mg of the petroleum extract was tested for spasmolytic activity using acetylcholine (Ach), 5-hydroxytryptamine (5—HT) and histamine as agonists as described below.

Guinea pigs weighing 200—300 g were killed by a blow on the head. The proximal ileum was excised and 2—3 cm length were cleaned and suspended in Krebs solution (NaCl 118.4, KCl 4.7, $CaCl_2$ 2.5, $MgSO_4$ 1.2, $KH_2PO_4$ 1.2, $NaHCO_3$ 25 and glucose 11.5 mMole/litre) at 37°C through which a mixture of 95% $O_2$ and 5% $CO_2$ was constantly bubbled. The lengths of ileum were set up to record longitudinal contractions insometrically. Log (dose) vs response curves were recorded to acetylcholine, 5-hydroxytryptamine and histamine from which $ED_{50}$ doses were taken and given repeatedly until constant responses were achieved. The antagonist *i.e.* the petroleum extract, was then dissolved in the minimum of dimethylsulphoxide and made up to a concentration of $10^{-4}$ g/ml with Krebs solution. This was added to the bath containing the ileum and left for 30 minutes. After thorough washing with Krebs solution to remove the residual antagonist the responses of the tissue to the agonists were then recorded and the precentage change calculated.

A control experiment was performed in exactly the same way except the antagonist was omitted. (This was essential since leaving an undosed tissue for 30 minutes often increases its sensitivity to exogenous agonist). The petroleum extract showed 100% inhibition to the three agonists.

4. Crude separation of the light petroleum extract

1.25 g of the petroleum extract was taken and chromatographed on a silica gel column (50 g) to investigate the components present and the degree of separation in view of a larger scale examination. This pilot study proved satisfactory and so 40 g of the petrol extract was placed on a column containing 1.2 kg silica gel mixed to a slurry with light petroleum b.r. 40—60°. 200 ml fractions were collected. The fractions were examined by TLC and combined as appropriate (see the drawing).

Solvent elution of the column and weights of the combined fractions are shown in Tables 1 and 2.

TABLE 1
Solvent elution of the column chromatography of the light petroleum extract

| Fraction numbers | Solvent |
|---|---|
| 1—24 | light petroleum b.r. 40°—60° |
| 25—36 | 1.0% v/v ethyl acetate in light petroleum |
| 37—50 | 2.5% v/v ethyl acetate in light petroleum |
| 51—65 | 5.0% v/v ethyl acetate in light petroleum |
| 66—95 | 7.5% v/v ethyl acetate in light petroleum |
| 96—112 | 10.0% v/v ethyl acetate in light petroleum |
| 113—118 | 25.0% v/v ethyl acetate in light petroleum |
| 119—166 | 50.0% v/v ethyl acetate in light petroleum |
| 167—174 | 75.0% v/v ethyl acetate in light petroleum |
| 175—180 | ethyl acetate |
| 181—185 | 1.0% v/v chloroform in ethyl acetate |
| 186—188 | 5.0% v/v chloroform in ethyl acetate |
| 189—192 | 10.0% v/v chloroform in ethyl acetate |
| 193—195 | 25.0% v/v chloroform in ethyl acetate |
| 196—201 | 50.0% v/v chloroform in ethyl acetate |
| 202—217 | chloroform |
| 218—237 | 10.0% v/v methanol in chloroform |
| 238 | 50.0% v/v methanol in chloroform |

TABLE 9
Weights of combined fractions from the column chromatography of the light petroleum extract

| Combined fractions (fraction numbers) | Weight (grams) | % of total (corrected to 1 decimal place) |
|---|---|---|
| 1—8 | 2.995 | 7.5 |
| 9—26 | 2.692 | 6.7 |
| 27—50 | 0.380 | 1.0 |
| 51—61 | 5.850 | 13.7 |
| 62—70 | 1.170 | 2.9 |
| 71—74 | 0.230 | 0.6 |
| 75—80 | 0.700 | 1.8 |
| 81—84 | 1.800 | 4.5 |
| 85—86 | 0.764 | 1.9 |
| 87—92 | 2.380 | 6.0 |
| 93—101 | 1.520 | 3.8 |
| 102—111 | 1.878 | 4.7 |
| 112—118 | 1.409 | 3.5 |
| 119—123 | 1.743 | 4.4 |
| 124—143 | 7.625 | 19.1 |
| 144—176 | 2.059 | 5.2 |
| 177—200 | 0.796 | 2.0 |
| 201—228 | 1.646 | 4.1 |
| 229—238 | 2.046 | 5.1 |
| | 39.633 | 98.5 |

5. Spasmolytic activity of the combined fractions from the light petroleum extract

The combined fractions from the petroleum extract were tested for spasmolytic activity as described in Part 3 above. The results are shown in Table 3.

TABLE 3

Spasmolytic activity of the combined fractions from the light petroleum extract, tested at $10^{-4}$ g/ml

| Fraction numbers | % inhibition of Ach | 5—HT | Histamine |
|---|---|---|---|
| 1—8 | 0 | 0 | 0 |
| 9—26 | 0 | 0 | 0 |
| 27—50 | 20 | 31 | 33 |
| 51—61 | 89 | 100 | 96 |
| 62—70 | 26 | 32 | 26 |
| 71—74 | 55 | 87 | 50 |
| 75—80 | 91 | 100 | 100 |
| 81—84 | 0 | 0 | 0 |
| 85—86 | 12 | 27 | 26 |
| 87—92 | 5 | 42 | 39 |
| 93—101 | 60 | 86 | 79 |
| 102—111 | 58 | 62 | 66 |
| 112—118 | 20 | 42 | 35 |
| 119—123 | 84 | 97 | 97 |
| 124—143 | 91 | 100 | 100 |
| 144—176 | 100 | 100 | 100 |
| 177—200 | 81 | 100 | 100 |
| 201—228 | 50 | 57 | 46 |

6. Extraction of tanacetum parthenium with chloroform

The plant materinl, 950 g, previously extracted with light petroleum b.r. 40—60°C was exhaustively extracted with chloroform in a Soxhlet apparatus for 9 days. The extract was evaporated to dryness under reduced pressure using a rotary evaporator to give a green oily residue weighing 46 g.

7. Spasmolytic activity of the chloroform extract

50 mg of the chloroform extract was tested for spasmolytic activity as before (see Part 3 above). 100% inhibition of the three agonists was obtained.

8. Crude separation of the chloroform extract

4.5 g of the chloroform extract was chromatographed on a silica gel column (120 g) to investigate the components present and the degree of separation in view of a larger scale examination. 100 ml fractions were taken and combined as appropriate with reference to TLC. Solvent elution of the column and weights of the combined fractions are shown in Tables 4 and 5.

TABLE 4
Solvent elution of the column chromatography of the chloroform extract

| Fraction numbers | Solvent |
|---|---|
| 1—12 | 50.0% v/v ethyl acetate in light petroleum |
| 13—16 | 75.0% v/v ethyl acetate in light petroleum |
| 17—27 | ethyl acetate |
| 28—32 | 10.0% v/v chloroform in ethyl acetate |
| 33—36 | 20.0% v/v chloroform in ethyl acetate |
| 37—40 | 50.0% v/v chloroform in ethyl acetate |
| 41—45 | chloroform |
| 46—54 | 10.0% v/v methanol in chloroform |
| 55—62 | 25.0% v/v methanol in chloroform |
| 63—68 | 50.0% v/v methanol in chloroform |

TABLE 5
Weights of combined fractions of the column chromatography of the chloroform extract

| Combined fractions (fraction numbers) | Weight (grams) | % of total (correction to 1 decimal place) |
|---|---|---|
| 1—2 | 0.429 | 9.5 |
| 3—4 | 0.417 | 9.3 |
| 5—6 | 0.171 | 3.8 |
| 7—8 | 0.097 | 2.2 |
| 9—12 | 0.201 | 4.5 |
| 13—18 | 0.362 | 8.1 |
| 19—22 | 0.273 | 6.1 |
| 23—25 | 0.410 | 9.2 |
| 26—39 | 0.264 | 5.9 |
| 40—54 | 0.940 | 20.9 |
| 55—68 | 0.805 | 17.9 |
| | 4.369 | 97.4 |

9. Spasmolytic activity of the combined fractions from the chloroform extract

The combined fractions from the chloroform extract were tested for spasmolytic activity as described in Part 3 above. The results are shown in Table 6. No fractions showed 100% antagonism to all three agonists and so were not investigated further with respect to pharmacological activity. The extract was fractionated however to isolate new compounds in the hope that these would be of help in the structural elucidation of the active ones from the light petroleum extract.

TABLE 6

Spasmolytic activity of the combined fractions from the chloroform extract, tested at $10^{-4}$ g/ml

| Fraction numbers | % inhibition of | | |
|---|---|---|---|
| | Ach | 5-HT | Histamino |
| 1— 2 | 9 | 0 | 26 |
| 3— 4 | 46 | 50 | 100 |
| 5— 6 | 91 | 94 | 93 |
| 7— 8 | 60 | 65 | 78 |
| 9—12 | 52 | 54 | 63 |
| 13—18 | 22 | 33 | 80 |
| 19—22 | 0 | 0 | 0 |
| 23—25 | 0 | 0 | 0 |
| 26—39 | 43 | 32 | 16 |
| 40—54 | 48 | 47 | 35 |
| 55—68 | 23 | 37 | 14 |

Studies on Fractions 144—176 (A) from the Light Petroleum Extract

Fractions 144—176 from the petroleum extract showed 100% inhibition of the three agonists Ach, 5-HT and histamine at $10^{-4}$ g/ml.

A further Separation of Fractions 144—176 (A)

These fractions (1.959 g) were chromatographed on a silica gel column (40 g). 100 ml fractions were taken and combined as appropriate with reference to TLC. Solvent elution of the column and weight of the combined fractions are shown in Tables 7 and 8.

TABLE 7

Solvent elution of column chromatography of fractions 144—176 (A) from the light petroleum extract.

| Fraction number | Solvent |
|---|---|
| A 1— 7 | hexane |
| A 8—25 | 10% v/v ethyl acetate in hexane |
| A 26—50 | 20% v/v ethyl acetate in hexane |
| A 51—65 | 40% v/v ethyl acetate in hexane |
| A 66—78 | 60% v/v ethyl acetate in hexane |
| A 79—85 | ethyl acetate |
| A 86 | chloroform |

TABLE 8

Weights of combined fractions of column chromatography of fractions 144—176 (A) from the light petroleum extract

| Fraction number | Weight (grams) | % total (corrected to 1 decimal place) |
|---|---|---|
| A 1— 7 | 0.022 | 1.1 |
| A 8— 9 | 0.014 | 0.7 |
| A 10—18 | 0.017 | 0.9 |
| A 19—24 | 0.048 | 2.5 |
| A 25—29 | 0.096 | 4.9 |
| A 30—34 | 0.211 | 10.8 |
| A 35—36 | 0.146 | 7.5 |
| A 37—38 | 0.102 | 5.2 |
| A 39—46 | 0.262 | 13.4 |
| A 47—52 | 0.177 | 9.0 |
| A 53—58 | 0.218 | 11.1 |
| A 59—65 | 0.267 | 13.6 |
| A 66—67 | 0.104 | 5.3 |
| A 68—72 | 0.125 | 6.4 |
| A 73—85 | 0.156 | 8.0 |
| | 1.965 | 100.2 |

B. Spasmolytic Activity of Fractions 144—176 (A)

Those fractions weighing more than 50 mg were tested for spasmolytic activity as described in Part 3 above. The results are shown in Table 9.

TABLE 9

Spasmolytic activity of fractions 144—176 (A) from the light petroleum extract, tested at $10^{-4}$ g/ml.

| Fraction number | % inhibition of | | |
|---|---|---|---|
| | Ach | 5-HT | Histamine |
| A 25—29 | 77 | 100 | 87 |
| A 30—34 | 60 | 48 | 55 |
| A 35—36 | 94 | 100 | 100 |
| A 37—38 | 97 | 98 | 97 |
| A 39—46 | 83 | 93 | 92 |
| A 47—52 | 100 | 100 | 100 |
| A 53—58 | 100 | 100 | 100 |
| A 59—65 | 100 | 100 | 100 |
| A 66—67 | 90 | 100 | 100 |
| A 68—72 | 87 | 90 | 92 |
| A 73—85 | 75 | 80 | 70 |

From Table 9, it can be seen that extracts from feverfew within the present invention which are obtained by extraction with a non-polar solvent have particularly high spasmolytic activity, which, we believe, is useful in the treatment of migraine. We expect, therefore, that at least those fractions showing 100% inhibition of all of the three agonists Ach, 5-HT and histamine will be effective against migraine. We expect also that these fractions would also be useful as anti-inflammatory agents and in the treatment of arthritic and bronchial complaints. As shown below these fractions all contain sesquiterpene lactones.

C Further Investigation of the Fractions Showing 100% Inhibition of the Three Agonists

(a) *Fractions A 59—65*

Since fractions A 59—65 weighed the most of the 100% active fractions this was investigated first.

It was decided to separate fractions A 59—65 by preparative TLC. 204 mg was placed on four 20 × 20 cm TLC plates (0.25 mm thickness) and developed in chloroform:methanol (98:2).

Eight bands were located under ultraviolet light (254 nm). The components in each band were extracted with chloroform in the usual way.

The major components of fractions A 59—65, *i.e.* band 6, crystallised from chloroform and methanol as fine needles (26 mg) of DJ140a, *8'α,[4'α,5'β-epoxy-1'β-(1β,10β-epoxy-4α-hydroxygermacr-11(13)-en-12, 6α-olactoyl-5β-yloxy)-germacra-10'(14'), 11'(13')-dien-12', 6'α-olactoyl]-8''α-(3α-hydroxy-3α,4α-dimethyl-2α-methylenepentanoyl)-4''α,5''β-epoxy-1''β, 6''α-dihydroxygermacra-10''(14''), 11''(13'')-dien-12''-oate (chrysanthemonin,* ), m.p. 211° (d); UV: $\lambda_{max}^{EtOH}$ (log ε) 210 (4.01) nm; Ir $\nu_{max}^{Solid\ film}$ 3470 (hydroxyl), 1760 (>C=O, γ-lactone, 1715 (>C=O, ester), 1665 (>C=C<, conjugated), 1640 (>C=C<), 1265

(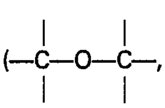,

epoxide) $cm^{-1}$; $^1H$ NMR: δ (400 MHz, $CDCl_3$), 1.07 (3H, *d*, J=7.0 Hz, H-5as or 6as), 1.21 (3H, *d*, J=7.0 Hz, H-6as or 5as), 1.32 (3H, *s*, H-15s), 1.33 (3H, *s*, H-7as), 1.43 (3H, *s*, H-15's), 1.44 (3H, *s*, H-15''s), 1.59 (3H), *s*, H-14s), 1.97 (1H, *dd*, J=8.0, 2.0 Hz, H-9'), 2.03 (1H, *m*, H-9''), 2.18 (1H, *d*, J=10.0 Hz, H-5), 2.38 (1H, *d*, J=9.5 Hz, H-5''), 2.42 (1H, *d*, J=11.5 Hz, H-5'), 2.58 (1H, *m*, H-3a), 2.82 (1H, *dd*, J=9.0, 9.0 Hz, H-7'), 2.89 (1H, *dd*, J=9.5, 9.5 Hz, H-7''), 2.99 (1H, *m*, H-1''), 3.08 (1H, *m*, H-7), 3.36 (1H, *s*, H-1), 3.99 (1H, *m*, H-1'), 4.01 (1H, *dd*, J=11.5, 9.0 Hz, H-6'), 4.09 (1H, *dd*, J=9.5, 9.5 Hz, H-6''), 4.13 (1H, *dd*, J=10.0, 10.0 Hz, H-6), 4.78 (1H, *d*, J=2.0 Hz, H-14'), 4.81 (1H, *d*, J=2.0 Hz, H-14''), 5.09 (1H, *d*, J=2.0 Hz, H-14'), 5.12 (1H, *d*, J=2.0 Hz, H-14''), 5.19 (1H, *m*, H-8'), 5.33 (1H, *d*, J=3.5 Hz, H-13a), 5.36 (1H, *m*, H-8''), 5.39 (1H, *d*, J=3.5 Hz, H-13''a), 5.94 (2H, *m*, H-8a), 6.05 (1H, *dd*, J=5.5, 1.0 Hz, H-13'a), 6.07 (1H, *d*, J=3.5 Hz, H-13b), 6.09 (1H, *dd*, J=5.5, 1.0 Hz, H-13'b), 6.13 (1H, *d*, J=3.5 Hz, H-13''b); MS: m/z (%, rel. int.), 381 (1.7), 367 (2.0), 273 (2.1), 261 (1.7), 259 (1.9), 247 (2.4), 246 (8.1),

245 (4.7), 243 (11.0), 229 (13.0), 228 (39.0), 226 (14.2), 213 (12.2), 202 (6.3), 201 (10.7), 200 (10.2), 199 (6.0), 198 (5.2), 197 (5.7), 185 (7.0), 183 (9.1), 173 (6.4), 169 (7.1), 167 (7.9), 157 (8.6), 156 (8.2), 97 (34.5), 95 (15.2), 94 (100.0), 83 (33.1), 71 (19.8), 43 (11.3).

(i) Hydrolysis of DJ140a

52 mg of DJ140a (combined with mother liquors) was dissolved in dioxan (5 ml) and a stoichiometric amount of $K_2CO_3$ (*i.e.* 245 microlitres of a solution containing 500 mg in 5 ml) was added. The reaction was monitored by TLC. After seven days with a gradual increase in temperature and addition of more base only starting material could be detected.

2 ml of a 2% solution of KOH in $H_2O$ was then added and the solution refluxed for 30 minutes. After normal work up the mixture was separated by preparative TLC using $CBCl_3$: MeOH (49:2) as developing solvent. More than twenty bands were located by ultraviolet light (254 nm) and spraying the edge of the plate with 20% v/v sulphuric acid and heating. The major component, located in band 2, was extracted with $CHCl_3$, DJ140a (*i.e.* the starting material) was located in band 3 and this was extracted and hydrolysed again using 2% w/v KOH solution and worked up as before. The organic solvent-soluble portion of this and the extract from band 2 of the original hydrolysis were identical on TLC. They were combined and purified by TLC using $CHCl_3$: MeOH (98:2) as developing solvent. The plate was developed three times. The major component was located in band 2 by spraying the edge of the plate with sulphuric acid as before and heating.

This component was further purified by TLC using $CHCl_3$: MeOH (98:2) as developing solvent. After extraction into $CHCl_3$ the material crystallised from $CHCl_3$ and hexane as colourless plates (5 mg) of DJ 140aH2, *4'α,5'β-epoxy-8'α-hydroxy-1'β-(1β,10β-epoxy-4α-hydroxygermacr-11(13)-en-12,6α-olactoyl-5β-yloxy)-germacra-10'(14'), 11'(13')-dien-12',6'α-olactone* ), m.p. 159° (d); UV: $\lambda_{max}^{EtOH}$ (log ε), 209 (3.99) nm; IR: $\nu_{max}^{Solid\ film}$, 3500 (OH), 1760 (>C=O, γ-lactone), 1665 (>C=C<, conjugated), 1640 (>C=C<), 1260

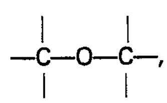

epoxide, $cm^{-1}$; $^1H$ NMR: δ (400 MHz, $CDCl_3$), 1.32 (3H, *s*, H-15s), 1.40 (3H, *s*, H-15's), 1.48 (1H, *m*, H-8), 1.55 (3H, *s*, H-14s), 1.77 (1H, *m*, H-9'), 2.06 (1H, *m*, H-9'), 2.23 (1H, *m*, H-8), 2.30 (1H, *d*, J=14.5 Hz), 2.44 (1H, *m*, H-7'), 2.46 (1H, *d*, J=11.5 Hz, H-5'), 2.59 (1H, *dd*, J=14.5, 5.0 Hz), 2.68 (1H, *d*, J=9.5 Hz, H-5), 3.01 (1H, *m*, H-8'), 3.20 (1H, *m*, H-7), 3.35 (1H, *s*, H-1), 3.92 (1H, *dd* J=11.5, 8.0 Hz, H-6'), 4.03 (1H, *m*, H-1'), 4.13 (1H, *dd*, J=9.5, 9.5 Hz, H-6), 4.91 (1H, *d*, J=1.5 Hz, H-14'), 5.15 (1H, *d*, J=1.5 Hz, H-14'), 5.35 (1H, *d*, J=3.5 Hz, H-13a), 5.89 (1H, *dd*, J=5.5, 0.7 Hz, H-13'a), 6.07 (1H, *dd*, J=5.5, 0.7 Hz, H-13'b), 6.09 (1H, *d*, J=3.5 Hz, H-13b); $^{13}C$ NMR: δ (100.6 MHz, $CDCl_3$), 15.10, 18.69 and 29.10 (C-14, C-15 and C-15'), 23.40, 33.35, 34.64, 38.09, 38.77, 65.04, and 66.08 (C-2, C-2', C-3', C-8, C-9 and C-9'), 43.24 and 44.31 (C-7 and C-7'), 50.72 (C-8'), 55.30, 61.39 and 73.27 (C-4, C-10 and C-4'), 50.86, 62.90, 65.66 and 68.88 (C-1, C-1', C-5 and C-5'), 75.35 and 79.95 (C-6 and C-6'), 118.00 and 118.64 (C-13 and C-13'), 135.69 (C-14'), 137.92 (C-10'), 140.98 (C-11), 170.60 and 178.64 (C-12 and C-12'). C-11' not seen: MS: m/z (%, rel.int.), 430 (1.1), 356 (1.3), 342 (1.3), 281 (2.7), 281 (1.0), 280 (4.3), 279 (12.9), 266 (1.4), 265 (7.1), 261 (1.3), 247 (2.7), 228 (5.0), 207 (8.9), 167 (15.4), 150 (10.5), 149 (100), 113 (5.3), 99 (6.1), 98 (5.4), 97 (6.4), 94 (9.7), 87 (5.5), 85.1 (11.1), 85.0 (30.6), 83.1 (7.8), 83.0 (46.0), 71 (18.4), 70 (10.5), 69 (15.2), 60 (5.6), 57 (50.7), 56 (16.9), 55 (22.9); Accurate mass measurements: Found 265.1449; $C_{15}H_{21}O_4$ requires 265.1440; Found: 279.1241; $C_{15}H_{19}O_5$ requires 279.1232.

(b) *Fractions A 53—58 and A 66-67*

These two sets of fractions were combined (0.236 g) and chromatographed on a silica gel column (10 g). 100 ml fractions were taken and combined as appropriate with reference to TLC. Solvent elution of the column and weights of the combined fractions are shown in Tables 10 and 11.

TABLE 10

Solvent elution of column chromatography of fractions A 53—58 and A 66-67 from the light petroleum extract

| Fraction number | Solvent |
|---|---|
| Aa 1—19 | 2.5% v/v chloroform in hexane |
| Aa 20—22 | 5% v/v chloroform in hexane |
| Aa 23—30 | 10% v/v chloroform in hexane |
| Aa 31—63 | 20% v/v chloroform in hexane |
| Aa 64—98 | 30% v/v chloroform in hexane |

TABLE 11

Weights of combined fractions of column chromatography of fractions A 53—58 and A 66—67 from the light petroleum extract

| Fraction number | Weight | % of total |
|---|---|---|
| Aa 1—46 | 0.007 | 3.0 |
| Aa 47—49 | 0.019 | 8.1 |
| Aa 50—63 | 0.039 | 16.5 |
| Aa 64—69 | 0.048 | 20.3 |
| Aa 70—76 | 0.030 | 12.7 |
| Aa 77—93 | 0.056 | 23.7 |
| Aa 94—98 | 0.028 | 11.9 |
| | 0.227 | 96.2 |

Fractions Aa 70—76 contained only one major component which crystallised from chloroform and methanol to give 14 mg of DJ140a, identical with that obtained from fractions A 59—65, above.

Fractions Aa 50—63 (0.039 g) were further separated by preparative TLC using chloroform:methanol (98:2) as the developing solvent. Three bands were located under ultra-violet light (254 nm) and their constituents extracted with chloroform. The major components of fractions Aa 50—63, *i.e.* in band 2, weighed 18 mg and crystallised from chloroform and hexane as colourless needles (8 mg) of DJ177b, *9,10-epoxy-3, 5-dihydroxyguia-4(15), 11(13)-dien-12, 6α-olactone (chrysanthemolide,* ), m.p. 116—117° (d); UV: $\lambda_{max}^{EtOH}$ (log ε) 215 (4.10) nm; IR: $\nu_{max}^{solid\ film}$, 3540 (OH), 1760 (>C=O, γ-lactone), 1665 (>C=C<, conjugated), 1640 (>C=C<), 1250

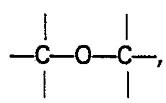

epoxide) $cm^{-1}$; $^1H$ NMR: δ (400 MHz, $CDCl_3$), 1.57 (3H, *s*, H-14s), 1.73 (2H, *m*, H-9s), 1.98 (2H, *m*, H-8s), 2.18 (2H, *m*, H-2s), 2.31 (1H, *dd*, J=13.5, 11.0 Hz, H-7), 2.81 and 3.97 (1H each, br ss, C-3 and C-5 O*H*s), 4.41 (1H, *m*, H-3), 4.88 (1H, *s*, H-15a), 5.21 (1H, *s*, H-15b), 5.23 (1H, *d*, J=11.0 Hz, H-6), 5.45 (1H, *d*, J=3.5 Hz, H-13a), 6.18 (1H, *d*, J=3.5 Hz, H-13b); MS: m/z (% rel. int.), 280 (2.0), 279 (2.6), 278 (0.2), 267 (3.4), 266 (5.5), 265 (16.8), 264 (40.8), 263 (17.9), 262 (6.2), 258 (4.9), 260 (8.0), 258 (4.9), 230 (22.0), 226 (10.5), 223 (7.2), 222 (18.2), 220 (10.7), 180 (18.6), 179 (11.9), 169 (10.9), 166 (13.2), 165 (18.0), 164 (14.7), 163 (14.6), 161 (10.4), 157 (20.4), 155 (14.1), 153 (10.7), 152 (19.2), 151 (21.9), 150 (24.7), 143 (24.0), 137 (32.9), 135 (21.1), 129 (20.1), 125 (25.8), 124 (30.1), 123 (37.4), 111 (43.4), 110 (45.4), 109 (46.6), 101 (54.0), 97 (68.1), 95 (74.3), 85 (58.9), 84 (65.7), 83 (100.0), 81 (64.0), 69 (68.7); Accurate mass measurement: Found 278.1150; $C_{15}H_{18}O_5$ requires 278.1154.

(i) Acetylation of DJ177b

4 mg of DJ177b was acetylated in the normal way using pyridine and acetic anhydride. After the usual work up spots for both starting material and a less polar product were present on TLC examination. The material was too little to be completely purified but the $^1H$ NMR spectrum of the crude product showed the following additional signals which may be assigned to the acetate product: δ, $CDCl_3$: 2.16 (3H, *s*, $CH_3CO_2$—) and 4.60 (1H, *m*, H-3).

(c) *Fractions A47—52*

Fractions 47—52 (0.123 g) were chromatographed on a silica gel column (10 g). 100 ml fractions were taken and combined as appropriate with reference to TLC. Solvent elution of the column and weight of the combined fractions are shown in Tables 12 and 13.

Fractions Ab 34—56 were further separated by preparative TLC using chloroform:methanol (98:2) as the developing solvent. Four bands were located under ultraviolet light (254 nm) and their components extracted with chloroform. The major component, *i.e.* contained in band 3, weighed 20 mg and crystallised from chloroform and *n*-pentane to give DJ179c. This material was a mixture of sesquiterpene lactones which resisted all attempts at purification. It was thus not investigated further.

TABLE 12
Solvent elution of column chromatography of fractions A47—52 from the light petroleum extract

| Fraction number | Solvent |
|---|---|
| Ab 1— 8 | 10% v/v chloroform in hexane |
| Ab 9—20 | 20% v/v chloroform in hexane |
| Ab 21—56 | 25% v/v chloroform in hexane |
| Ab 57 | chloroform |

TABLE 13
Weights of the combined fractions of column chromatography of fractions A47—52 from the light petroleum extract

| Fraction number | Weight (grams) | % total |
|---|---|---|
| Ab 1—19 | 0.015 | 12.2 |
| Ab 20—23 | 0.004 | 3.3 |
| Ab 24—33 | 0.039 | 31.7 |
| Ab 34—56 | 0.053 | 43.0 |
| Ab 57 | 0.007 | 5.7 |
| | 0.118 | 95.9 |

The components in band 1 (11 mg) were further purified by preparative TLC. The plate was run seven times with chloroform: methanol (98:2) as the developing solvent. Four bands were located under ultraviolet light (254 nm) and their components extracted with chloroform. The components contained in band 1 of the latter plate weighed 6 mg and crystallised from chloroform and *n*-pentane to give DJ179a1. This material was possibly also a mixture of sesquiterpene lactones despite showing only one spot on TLC investigation. Signals were seen in the $^1H$ NMR spectrum which could be assigned to reynosin, chrysartemin B and perhaps a dihydro-derivative of reynosin. The paucity of material precluded further study at this stage but the further possibility that of the material's being a trimeric compound rather like DJ140a cannot be discounted.

11 Studies on Fractions 124—143 (B) from the Light Petroleum Extract

Fractions 124—143 from the petroleum extract showed 100% inhibition of the two agonists 5-HT and histamine, and 91% inhibition of Ach.

A Further Separation of Fractions 124—143 (B)

These fractions (7.525 g) were chromatographed on a silica gel column (150 g). 100 ml fractions were taken and combined as appropriate with reference to TLC. Solvent elution of the column and weight of the combined fractions are shown in Tables 14 and 15.

TABLE 14
Solvent elution of column chromatography of fractions 124—143 (B) from the light petroleum extract

| Fraction number | Solvent |
|---|---|
| B 1— 27 | hexane |
| B 28— 45 | 1% v/v chloroform in hexane |
| B 46— 51 | 2% v/v chloroform in hexane |
| B 52— 60 | 5% v/v chloroform in hexane |
| B 61— 67 | 10% v/v chloroform in hexane |
| B 68— 80 | 20% v/v chloroform in hexane |
| B 81—136 | 40% v/v chloroform in hexane |
| B 137—149 | 60% v/v chloroform in hexane |
| B 150—170 | 75% v/v chloroform in hexane |
| B 171—194 | chloroform |
| B 195—203 | 90% v/v chloroform in methanol |
| B 204 | 50% v/v chloroform in methanol |

TABLE 15
Weights of combined fractions of column chromatography of fractions 124—143 (B) from the light petroleum extract.

| Fraction number | Weight (grams) | % of total |
|---|---|---|
| B 1— 9 | 0.011 | 0.1 |
| B 10— 90 | 0.040 | 0.5 |
| B 91—106 | 0.025 | 0.3 |
| B 107—111 | 2.484 | 33.0 |
| B 112—115 | 1.537 | 20.4 |
| B 116—122 | 0.490 | 6.5 |
| B 123—130 | 0.316 | 4.2 |
| B 131—147 | 0.252 | 3.3 |
| B 148—163 | 0.454 | 6.0 |
| B 164—171 | 0.200 | 2.6 |
| B 172—196 | 0.810 | 10.8 |
| B 197—198 | 0.682 | 9.1 |
| B 199 | 0.078 | 1.0 |
| B 200 | 0.083 | 1.1 |
| B 201—204 | 0.048 | 0.6 |
| | 7.510 | 99.6 |

B Spasmolytic Activity of Fractions 124—143 (B)

Those fractions weighing more than 50 mg were tested for spasmolytic activity as described in Part 3 above. The results are shown in Table 16.

TABLE 16

Spasmolytic activity of fractions 124—143 (B) from the light petroleum extract, tested at $10^{-4}$ g/ml

| Fraction number | % inhibition of | | |
|---|---|---|---|
| | Ach | 5-HT | Histamine |
| B 107—111 | 100 | 100 | 100 |
| B 112—115 | 100 | 100 | 100 |
| B 116—122 | 95 | 100 | 100 |
| B 123—130 | 82 | 100 | 98 |
| B 131—147 | 78 | 100 | 100 |
| B 148—163 | 51 | 88 | 97 |
| B 164—171 | 22 | 29 | 90 |
| B 172—196 | 3 | 44 | 100 |
| B 197—198 | 16 | 58 | 33 |
| B 199 | 31 | 72 | 65 |
| B 200 | 27 | 42 | 20 |

C Further Investigation of the Fractions Showing 100% Inhibition of the three Agonists

Fractions B 107—111 showed 100% inhibition of all three agonists. The fractions (2.434 g) were chromatographed on a silica gel column (120 g). 100 ml fractions were taken and combined as appropriate with reference to TLC. Solvent elution of the column and weight of the combined fractions are shown in Tables 17 and 18.

TABLE 17

Solvent elution of the column chromatography of fractions B 107—111 from the light petroleum extract

| Fraction number | Solvent |
|---|---|
| Ba 1— 5 | 10% chloroform in hexane |
| Ba 6—11 | 20% chloroform in hexane |
| Ba 12—70 | 30% chloroform in hexane |
| Ba 71—73 | chloroform |

TABLE 18
Weights of combined fractions of column chromatography of fractions B 107—111 from the light petroleum extract

| Fraction number | Weight (grams) | % of total |
|---|---|---|
| Ba 1—20 | 0.112 | 4.6 |
| Ba 21—32 | 0.465 | 19.1 |
| Ba 33 | 0.057 | 2.3 |
| Ba 34—55 | 1.421 | 58.4 |
| Ba 56—58 | 0.128 | 5.3 |
| Ba 59—68 | 0.130 | 5.3 |
| Ba 69—70 | 0.094 | 3.9 |
| Ba 71—73 | 0.030 | 1.2 |
| | 2.437 | 100.1 |

The major component of fractions B107—111, *i.e.* contained in subfractions Ba 34—55, crystallised from chloroform and hexane to give parthenolide (478 mg) as colourless plates, m.p. 113—114°; UV: $\lambda_{max}^{EtOH}$ (log ε) 213 (4.20) nm; IR: $\nu_{max}^{KBr}$ 1760 (>C=O, γ-lactone), 1660 (>C=C<, conjugated), 1640 (>C=C<), 1250 (

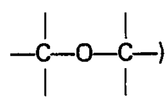

) cm$^{-1}$; $^1$H NMR: δ (400 MHz, $CDCl_3$), 1.32 (3H, *s*, H-15s), 1.73 (3H, *s*, H-14s), 2.81 (1H, *d*, J=8.5Hz, H-5), 2.81 (1H, *m*, H-7), 3.87 (1H, *dd*, J=8.5, 8.5 Hz, H-6), 5.23 (1H, *dd*, J=12.5, 3.0 Hz, H-1), 5.64 (1H, *d*, J=3.8 Hz, H-13a), 6.35 (1H, *d*, J=3.8 Hz, H-13b); $^{13}$C NMR: δ (100.6 MHz, $CDCl_3$), 16.97, 17.24 (C-14 and C-15), 24.11, 30.64 (C-8 and C-3), 36.35, 41.19 (C-2 and C-9), 47.65 (C-7), 61.45 (C-5), 66.36 (C-4), 82.38 (C-6), 121.08 (C-1), 125.24 (C-13), 134.51 (C-10), 139.21 (C-11), C-12 not detected; MS: m/z (%, rel. int.), 248 (1.5), 233 (1.1), 230 (8.1), 190 (9.3), 107 (8.4), 105 (12.2), 95 (8.9), 91 (13.6), 81 (12.4), 79 (11.7), 77 (9.2), 67 (9.6), 58 (24.8), 55 (12.0), 53 (17.3), 43 (100.0).

This material was identical in all respects with an authentic sample supplied by F. Sorm.

12 Studies on Fractions 75—80 (C) from the Light Petroleum Extract

Fractions 75—80 (C) from the petroleum extract showed 100% inhibition of the two agonists, 5-HT and histamine, and 91% inhibition of Ach.

A Further Separation of Fractions 75—80 (C)

0.65 g were chromatographed on a column of silica gel (60 g). 100 ml fractions were collected and combined as appropriate with reference to TLC. The weights of the combined fractions and solvent elution of the column are given in Tables 19 and 20.

TABLE 19
Solvent elution of column chromatography of fractions 75—80 (C) from the light petroleum extract

| Fraction number | Solvent |
|---|---|
| C 1— 3 | 5% chloroform in hexane |
| C 4—12 | 10% chloroform in hexane |
| C 13—29 | 20% chloroform in hexane |
| C 30—33 | 40% chloroform in hexane |
| C 34 | chloroform |

TABLE 20

Weights of combined fractions of column chromatography of fractions 75—80 (C) from the light petroleum extract

| Fraction number | Weight (grams) | % total |
|---|---|---|
| C 1—15 | 0.020 | 3.1 |
| C 16—19 | 0.036 | 5.5 |
| C 20—24 | 0.446 | 68.6 |
| C 25—26 | 0.044 | 6.8 |
| C 27—39 | 0.103 | 15.8 |
| | 0.649 | 99.8 |

All these subfractions were devoid of spasmolytic activity at a concentration of $10^{-4}$ g/ml and so were not investigated further.

13 Studies on Fractions 177—200 (D) from the Petroleum Extract

Fractions 177—200 from the petroleum extract showed 100% inhibition of the two agonists 5-HT and histamine, and 81% inhibition of Ach.

A Further Separation of Fractions 177—200 (D)

These fractions (0.729 g) were chromatographed on a silica gel column (30 g). 100 ml fractions were taken and combined as appropriate with reference to TLC. Solvent elution of the column and weights of the combined fractions are shown in Tables 21 and 22.

TABLE 21

Solvent elution of column chromatography of fractions 177—200 (D) from the light petroleum extract

| Fraction number | Solvent |
|---|---|
| D 1—12 | 25% v/v ethyl acetate in hexane |
| D 13—17 | 40% v/v ethyl acetate in hexane |
| D 18—24 | 60% v/v ethyl acetate in hexane |
| D 25—31 | 80% v/v ethyl acetate in hexane |
| D 32—36 | ethyl acetate |
| D 37—40 | 10% v/v chloroform in ethyl acetate |
| D 41—45 | 30% v/v chloroform in ethyl acetate |

TABLE 22

Weights of combined fractions of column chromatography of fractions 177—200 (D) from the light petroleum extract.

| Fraction number | Weight (grams) | % of total |
|---|---|---|
| D 1— 3 | 0.037 | 5.1 |
| D 4— 8 | 0.090 | 12.3 |
| D 9—10 | 0.049 | 6.7 |
| D 11—15 | 0.088 | 12.1 |
| D 16—20 | 0.240 | 32.9 |
| D 21—26 | 0.160 | 21.9 |
| D 27—45 | 0.070 | 9.6 |
| | Total=0.734 | 100.6 |

B Spasmolytic Activity of Fractions 177—200 (D)

Those fractions weighing more than 50 mg were tested for spasmolytic activity as described in Part 3 above. The results are as shown in Table 23.

TABLE 23

Spasmolytic activity of fractions 177—200 (D) from the light petroleum extract, tested at $10^{-4}$ g/ml

| Fraction number | % inhibition of | | |
|---|---|---|---|
| | Ach | 5-HT | Histamine |
| D 1— 4 | 64 | 96 | 91 |
| D 4— 8 | 81 | 92 | 93 |
| D 11—15 | 97 | 100 | 100 |
| D 16—20 | 100 | 100 | 100 |
| D 21—26 | 100 | 100 | 100 |
| D 27—45 | 47 | 52 | 67 |

C Further Investigation of the Fractions Showing 100% Inhibition of the Agonists

(a) *Fractions D16—20*

Fractions D 16—20 (0.175g) were further separated by preparative TLC using chloroform:methanol (95:5) as developing solvent. The plates were run three times. Three bands were located under ultraviolet light (254 nm) and their components extracted with chloroform. The major component, *i.e.* in band 1, weighed 59 mg and crystallised from chloroform and methanol as colourless plates (26 mg) of DJ 154a, m.p. 248°; UV: $\lambda_{max}^{EtOH}$ (log ε) 208 (3.98) nm; IR: $\nu_{max}^{KBr}$ 3420 (OH), 1760 (>C=O, γ-lactone), 1675 (>C=C<, conjugated), 1260

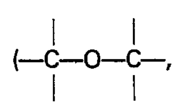

epoxide) $cm^{-1}$; $^1H$ NMR: δ (400 MHz, $CDCl_3$), 1.15 (3H, *s*, H-15s), 1.58 (3H, *s*, H-14s), 2.87 (1H, *d*, J=11.0 Hz, H-5), 3.30 (1H, *m*, H-7), 3.32 (1H, *d*, J=1.3 Hz, H-3), 3.57 (1H, *d*, J=1.3 Hz, H-2), 4.09 (1H, *dd*, J=11.0, 10.0 Hz, H-6), 5.54 (1H, *d*, J=3.5 Hz, H-13a), 6.19 (1H, *d*, J=3.5 Hz, H-13b); MS: m/z (%. rel. int.) 279 (0.7), 278 (2.1),

263 (2.3), 262 (4.7), 260 (3.1), 249 (3.1), 231 (6.3), 217 (8.6), 203 (9.4), 189 (12.5), 175 (15.6), 165 (26.6), 151 (40.6), 149 (23.4), 147 (28.9), 123 (26.6), 121 (33.6), 112 (88.3), 109 (42.2), 97 (35.9), 95 (93.8), 91 (41.4), 85 (47.7), 83 (44.5), 81 (36.7), 79 (38.3), 77 (35.9), 71 (78.1), 69 (52.3), 67 (49.2), 57 (93.8), 55 (71.1), 53 (75.8), 43 (100.0). Lit. m.p. 250° This material was identical in all respects with literature data on chrysartomin A.

(b) *Fractions D 21—26*

Fractions D21—26 (0.110 g) were further separated by preparative TLC using chloroform:methanol (95:5) as developing solvent. The plates were run three times. Three bands were located under ultraviolet light (254 nm) and by spraying the edge of the plate with $H_2SO_4$ and heating. Their components were extracted with chloroform.

The major component of fractions D 21—26, *i.e.* in band 2, crystallised from chloroform and hexane to give colourless needles (16 mg) of DJ156a, *4α,10β-dihydroxy-1β,5α-gui-11(13)-en-12,6α-olactone (partholide,* ) m.p. 154°; UV: $\lambda_{max}^{EtOH}$ (log ε) 208 (3.95) nm; IR: $\nu_{max}^{Solid\ film}$, 3490 (OH), 1750 (>C=O, γ-lactone), 1660 (>C=C<, conjugated), 1640 (>C=C<); $^1$H NMR: δ (400 MHz, $CDCl_3$), 1.25 (3H, *s*, H-14s), 1.35 (3H, *s*, H-15s), 1.47 (1H, *m*, H-8), 1.62 (2H, *m*, H-2s), 1.67—2.04 (6H, br *m*, H-3s, H-9s, 2 × OHs), 2.16 (1H, *m*, H-8), 2.39 (1H, *dd*, J=12.3, 12.3 Hz, H-5), 2.63 (1H, *m*, H-1), 2.70 (1H, *m*, H-7), 4.24 (1H, *dd*, J=12.3, 9.5Hz, H-6), 5.54 (1H, *d*, J=3.5 Hz, H-13a), 6.25 (1H, *d*, J=3.5 Hz, H-13b); $^{13}$C NMR: δ (100.6 MHz, $CDCl_3$), 23.51, 24.25 (C-14 and C-15), 25.01, 25.33 (C-2 and C-8), 39.34, 43.77 (C-3 and C-9), 47.22 (C-7), 49.78 (C-1), 55.35 (C-5), 74.72 (C-4), 79.88 (C-10), 82.74 (C-6), 120.37 (C-13), 138.45 (C-11), 169.37 (C-12); MS: m/z (% rel.int.), 266 (0.4), 265 (0.9), 264 (1.8), 263 (1.8), 262 (3.6), 261 (2.7), 260 (4.5), 249 (3.0), 248 (6.0), 247 (7.2), 246 (18.0), 245 (6.3), 244 (13.5), 232 (4.5), 231 (15.3), 230 (10.0), 229 (9.0), 228 (19.8), 215 (12.6), 213 (18.9), 203 (22.5), 191 (22.5), 190 (27.0), 188 (24.3), 175 (24.3), 173 (22.5), 159 (24.3), 105 (45.0), 95 (44.6), 93 (44.1), 91 (59.5), 83 (85.6), 81 (51.4), 79 (49.5), 71 (42.3), 69 (47.7), 57 (59.5), 55 (91.9), 43 (100.0); Accurate mass measurement: Found: 264.1364; $C_{15}H_{20}O_4$ requires 264.1362.

14 Studies on Fractions 119—123 (E) from the Light Petroleum Extract

Fractions 119—123 from the petroleum extract showed 97% inhibition of 5—HT and histamine and 84% inhibition of Ach.

A Further Separation of Fractions 119—123 (E)

These fractions (1.583 g) were chromatographed on a silica gel column (30 g). 100 ml fractions were taken and combined as appropriate with reference to TLC. Solvent elution of the column and weights of the combined fractions are shown in Tables 24 and 25.

TABLE 24

Solvent elution of column chromatography of fractions 119—123 (E) from the light petroleum extract

| Fraction number | Solvent |
|---|---|
| E 1— 2 | hexane |
| E 3— 5 | 10% v/v chloroform in hexane |
| E 6—31 | 20% v/v chloroform in hexane |
| E 32 | chloroform |

TABLE 25
Weights of combined fractions of column chromatography of fractions 119—123 (E) from the light petroleum extract

| Fraction number | Weight (grams) | % of total |
|---|---|---|
| E 1—13 | 0.010 | 0.6 |
| E 14 | 0.007 | 0.4 |
| E 15 | 0.058 | 3.7 |
| E 16—18 | 0.450 | 28.4 |
| E 19—21 | 0.201 | 12.7 |
| E 22—31 | 0.148 | 9.3 |
| E 32—36 | 0.251 | 15.9 |
| E 37—38 | 0.064 | 4.0 |
| E 39 | 0.395 | 25.0 |
| | 1.584 | 100.0 |

B Spasmolytic Activity of Fractions 119—123 (E)

Those fractions weighing more than 50 mg were tested for spasmolytic activity as described in Part 3 above. The results are shown in Table 26.

TABLE 26
Spasmolytic activity of fractions 119—123 (E) from the light petroleum extract, tested at $10^{-4}$ g/ml

| Fraction number | % inhibition of | | |
|---|---|---|---|
| | Ach | 5-HT | Histamine |
| E 15 | 67 | 97 | 91 |
| E 16—18 | 57 | 96 | 89 |
| E 19—21 | 85 | 100 | 100 |
| E 22—31 | 28 | 100 | 92 |
| E 32—36 | 85 | 100 | 98 |
| E 37—38 | 56 | 100 | 100 |
| E 39 | 78 | 100 | 91 |

C Further Investigation of the Fractions showing 100% Inhibition of the Agonists

Fractions E 15—21 were combined and purified by TLC using chloroform : hexane (75:25) as developing solvent. The major component was extracted and identified as parthenolide by chromatographic comparison with an authentic specimen and by spectroscopic means.

As can be seen from the above, extraction of feverfew with a non-polar solvent can provide fractions having high spasmolytic activity, from which fractions pure sesquiterpene lactones can be obtained.

Compounds 140a, 156a and 177b which we believe have not been previously disclosed in the literature have the following mobilities in thin layer chromatography.

| Compound | Solvent | $R_f$ |
|---|---|---|
| 140a | chloroform/methanol (98/2) — developed once | 0.49 |
| 156a | chloroform/methanol (95/5) — run three times | 0.35 |
| 177b | chloroform/methanol (98/2) — developed once | 0.34 |

Preparation of Capsules

Example 1

Powdered feverfew leaf and soya bean oil are mixed in the following proportions:—

| | |
|---|---|
| Powdered feverfew leaf | 10.0 kg |
| Soya Bean Oil | 100.0 kg |

The mixture is allowed to stand for 14 days with stirring for extraction of the active ingredients and then stabilized by:—

| | |
|---|---|
| Lecithin | 2.2 kg |
| Hydrogenated Fats | 30.0 kg |
| Beeswax | 10.0 kg |

The mixture is encapsulated into a soft shell capsule or hard shell capsule, representing a dose of 25 mg of dried leaf. This fills into a 7.5 oval soft shell capsule or a number 2 hard shell capsule with a target fill weight of 380.5 mg.

In an alternative method, the mixture is strained and the resulting macerate, encapsulated as the oily extract, is stabilised with one or more of the above described suspending agents, into soft shell or two piece hard shell gelatin capsules.

Example 2

Powdered feverfew leaf and cod liver oil are mixed in the following proportions:—

| | |
|---|---|
| Feverfew Dry Leaf | 10.0 kg |
| Cod Liver Oil | 100.0 kg |

The mixture is allowed to stand for 14 days for extraction of the active ingredients.

The resultant mixture is filtered and the filtrate encapsulated at a fill weight of 250 mg per unit dose, to represent approximately 25 mg of dried leaf in a soft shell or two piece hard shell capsule.

In an alternative method the suspension or macerate is mixed with surfactive agents or mixtures thereof, such as polyethoxy fatty acid sorbitan esters, mono and di glycerides or acetylated glycerides, to form a self emulsifying system.

The resultant formulation is encapsulated into a soft shell or two piece hard shell capsule.

It is predicted that mixtures and dosages will enhance absorption of the oily active principles via the lymphatic system.

Example 3

The following are mixed:—

| | |
|---|---|
| Feverfew dry leaf macerate (as above) | 100.0 kg |
| Polyethoxy sorbitan monooleate | 37.0 kg |
| Mono and di glycerides blend | 29.0 kg |

These are mixed and encapsulated at a dosage fill weight of 415 mg into soft shell or two piece hard shell capsule, representing approximately 25 mg of dried feverfew leaf.

The soft shell or two piece hard shell gelatin capsule may be used via the oral or rectal route.

They may be treated to retard disintegration or absorption by the use of gastro resistant coatings, such as hydroxymethyl propyl cellulose or the content may be mixed with polymeric matrix materials as known to the pharmaceutical industry, to release the active principles at a controlled rate.

**Claims for the Contracting States: DE FR IT NL SE CH BE LU LI**

1. A spasmolytically active composition for use in the treatment of a migraine, asthmatic, bronchial or arthritic condition, which composition comprises a sesquiterpene lactone — containing extract from the plant *Tanacetum parthenium*, which extract is dissolved in an oil.

2. A spasmolytically active composition according to claim 1, for use in the treatment of migraine.

3. A spasmolytically active composition according to claim 1, comprising particles of the dried plant *Tanacetum parthenium* suspended in the said oil, the oil serving to extract the sesquiterpene lactone from the dried plant.

4. A spasmolytically active composition according to claim 1, claim 2 or claim 3, wherein the oil is a saturated or unsaturated long chain hydrocarbon or fatty acid, a vegetable or animal oil, or a polyoxyethylated, reconstituted glyceride or ester derivative of the vegetable or animal oil.

5. A spasmolytically active composition according to claim 4, wherein the oil is coconut oil, soybean oil or fish oil.

6. A spasmolytically active composition according to any preceding claim which additionally contains a pharmaceutically acceptable excipient.

7. A spasmolytically active composition according to any one of the preceding claims, which is suitable for administration either orally, rectally, parenterally or by inhalation.

8. A spasmolytically active composition according to claim 7, which is in the form of a tablet, capsule or liquid suspension.

9. A spasmolytically active composition according to claim 8, which is in the form of an encapsulated preparation comprising particles of the dried plant *Tanacetum parthenium* suspended in an oil, the oil serving to extract the sesquiterpene lactone from the dried plant.

10. A spasmolytically active composition according to any preceding claim, which additionally contains at least one known antiarthritic agent.

11. A spasmolytically active composition according to any one of claims 1 to 9, which additionally contains at least one known bronchodilator, antihistamine or anti-infective agent.

12. A spasmolytically active composition according to any one of claims 1 to 9, which further contains one or more known anti-migraine ingredients, analgesics and/or anti-emetics.

13. Use of a sesquiterpene lactone for the manufacture of a spasmolytically active medicament for the treatment of a migraine, asthmatic, bronchial or arthritic condition, which sesquiterpene lactone is derived from the plant *Tanacetum parthenium* by a process comprising extraction of the plant with a non-polar organic solvent as at least a first extractant.

14. Use according to claim 13, wherein the non-polar solvent is light petroleum, hexane or chloroform.

15. Use according to claim 14, wherein the non-polar solvent is an oil.

16. Use according to claim 15, wherein the oil is a saturated or unsaturated long chain hydrocarbon or fatty acid, a vegetable or animal oil or a polyoxyethylated, reconstituted glyceride or ester of the vegetable or animal oil.

17. Use according to claim 16, wherein the oil is coconut oil, soybean oil or fish oil.

18. Use according to any one of claims 13 to 17, wherein the extract contains a plurality of sesquiterpene lactones.

19. Use according to any one of claims 13 to 18, wherein the or at least one sesquiterpene lactone has an α-methylene substituent on the lactone ring.

20. Use according to claim 19, wherein the sesquiterpene lactone is a germacranolide which is parthenolide of the formula

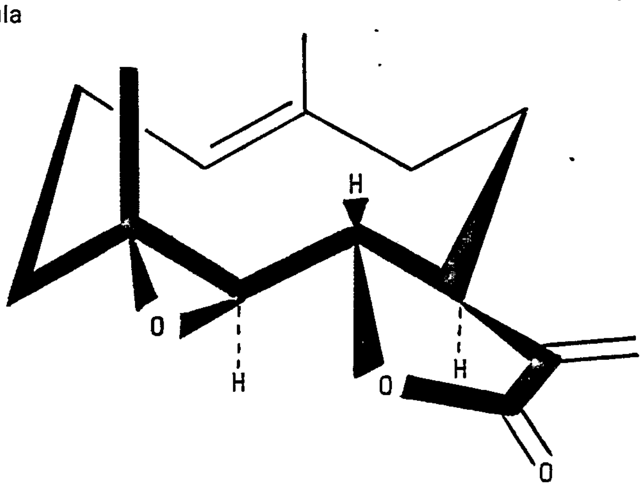

21. Use according to claim 19, wherein the sesquiterpene lactone is a germacranolide which contains at least two germacrane nucleii in its molecule.

22. Use according to claim 21, wherein the germacranolide has the formula

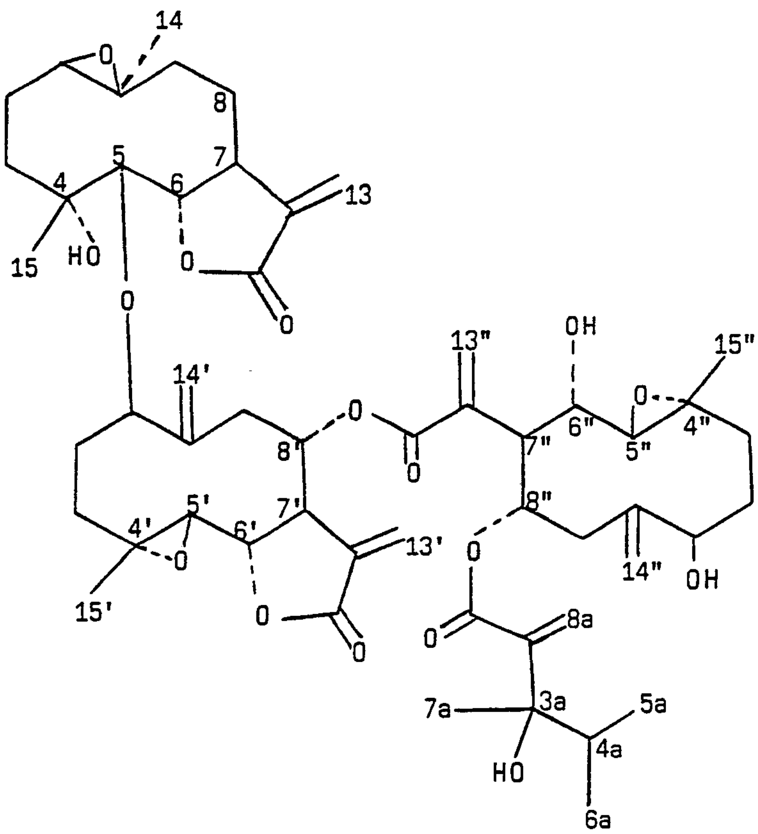

(5)

23. Use according to claim 13, wherein the said process comprising the extraction of the plant further comprises hydrolysis of the germacranolide (5), given and defined in claim 22, to provide a compound having the formula

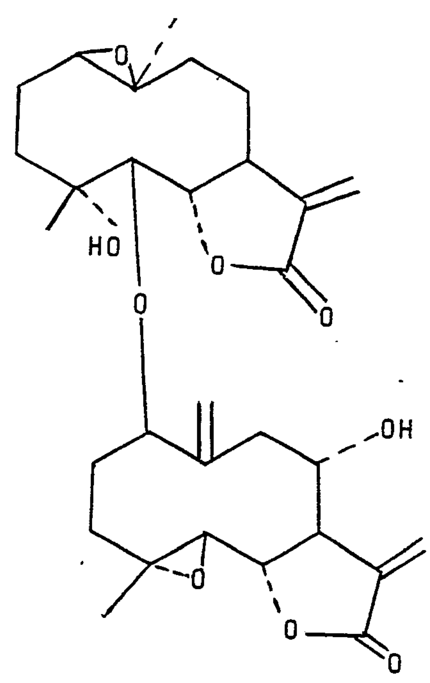

(6)

24. Use according to claim 19, wherein the sesquiterpene lactone is a guaianolide which has the formula

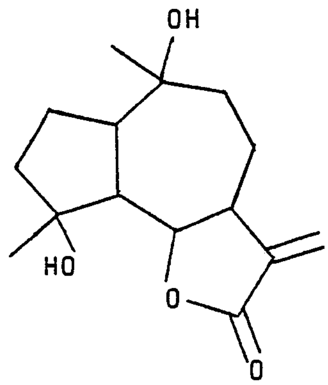

(3)'

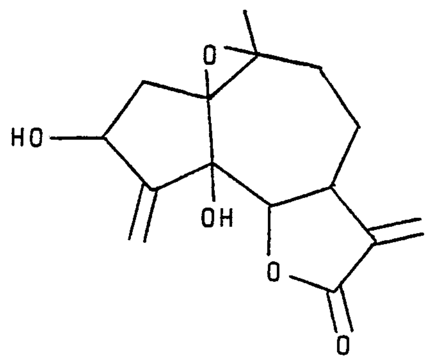

(4)

25. Use according to any one of claims 13 to 24 for the treatment of migraine.

26. A sesquiterpene lactone derived from the plant *Tanacetum parthenium* and comprising a germacranolide having the formula

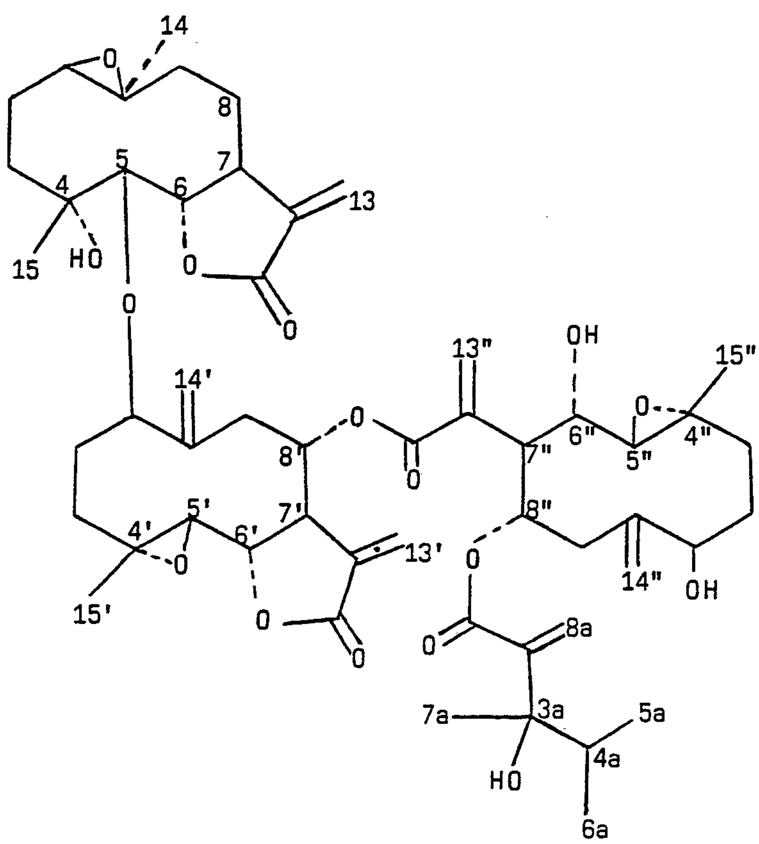

(5)

or

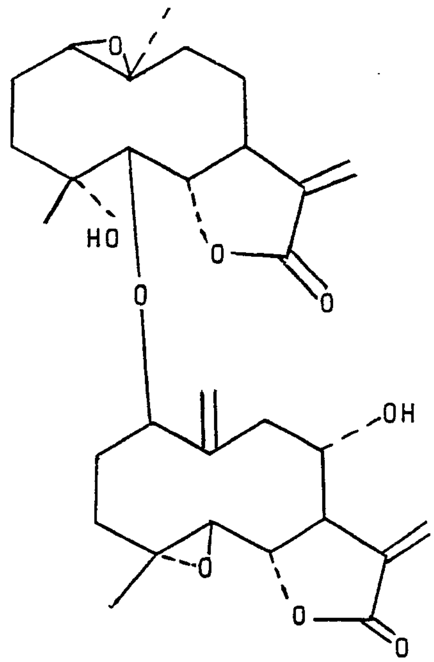

(6)

27. A sesquiterpene lactone derived from the plant *Tanacetum parthenium* which is a guaianolide having the formula

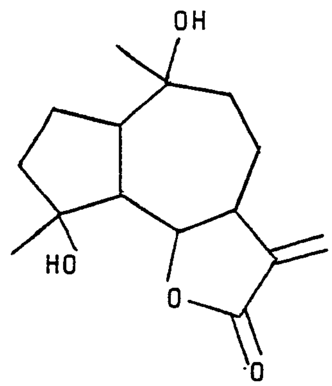

(3)'

or

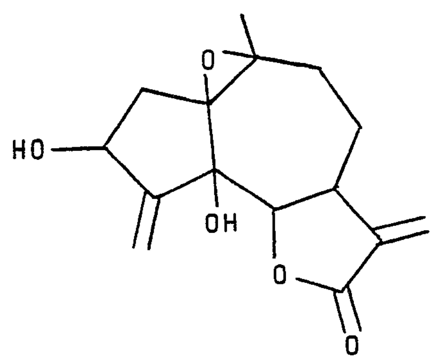

(4)

28. A sesquiterpene lactone according to claim 26 or 27 which is essentially pure.

29. A spasmolytically active composition for use in the treatment of migraine, asthmatic, bronchial or arthritic condition comprising a sesquiterpene lactone according to any one of claims 26 to 28 and a pharmaceutically accetable excipient.

30. A method of preparing a sesquiterpene lactone — containing extract for phamaceutical use, which method includes treating *Tanacetum parthenium* with a first non-polar solvent, evaporating the solvent and subjecting the resulting crude extract to chromatography using a second non-polar solvent as at least a first eluate.

**Claims for the Contracting State: AT**

1. A method of preparing a spasmolytically active composition for use in the treatment of a migraine, asthmatic, bronchial or arthritic condition, which method comprises treating the plant *Tanacetum parthenium* with an oil in which at least one said sesquiterpene lactone is soluble and allowing the said at least one sesquiterpene lactone to dissolve in the oil.

2. A method according to claim 1, comprising suspending particles of the dried plant *Tanacetum parthenium* in the said oil.

3. A method according to claim 1 or claim 2, wherein the oil is a saturated or unsaturated long chain hydrocarbon or fatty acid, a vegetable or animal oil, or a polyoxyethylated, reconstituted glyceride or ester derivative of the vegetable or animal oil.

4. A method according to claim 3, wherein the oil is coconut oil, soybean oil or fish oil.

5. A method of preparing a sesquiterpene lactone-containing extract for pharmaceutical use which method comprises treating the plant *Tanacetum parthenium* with a first non-polar organic solvent in which at least one said sesquiterpene lactone is soluble, evaporating the solvent and subjecting the crude extract to chromatography using a second non-polar solvent as at least a first eluate.

6. A method according to claim 5, wherein the extract contains a plurality of sesquiterpene lactones.

7. A method according to claim 5 or claim 6, wherein the non-polar solvent is light petroleum, hexane, chloroform or an oil.

8. A method according to claim 5, claim 6 or claim 7, wherein the or a said sesquiterpene lactone has an α-methylene substituent on the lactone ring.

9. A method according to claim 8, wherein the or a said sesquiterpene lactone is a germacranolide which is parthenolide of the formula

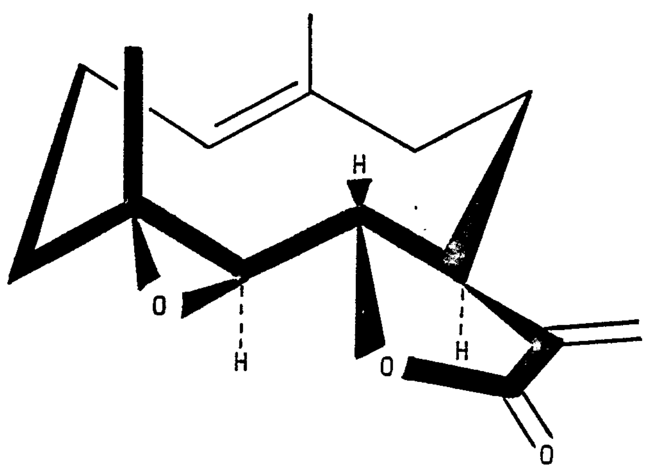

10. A method according to claim 8, wherein the or a said sesquiterpene lactone comprises a germacranolide having the formula

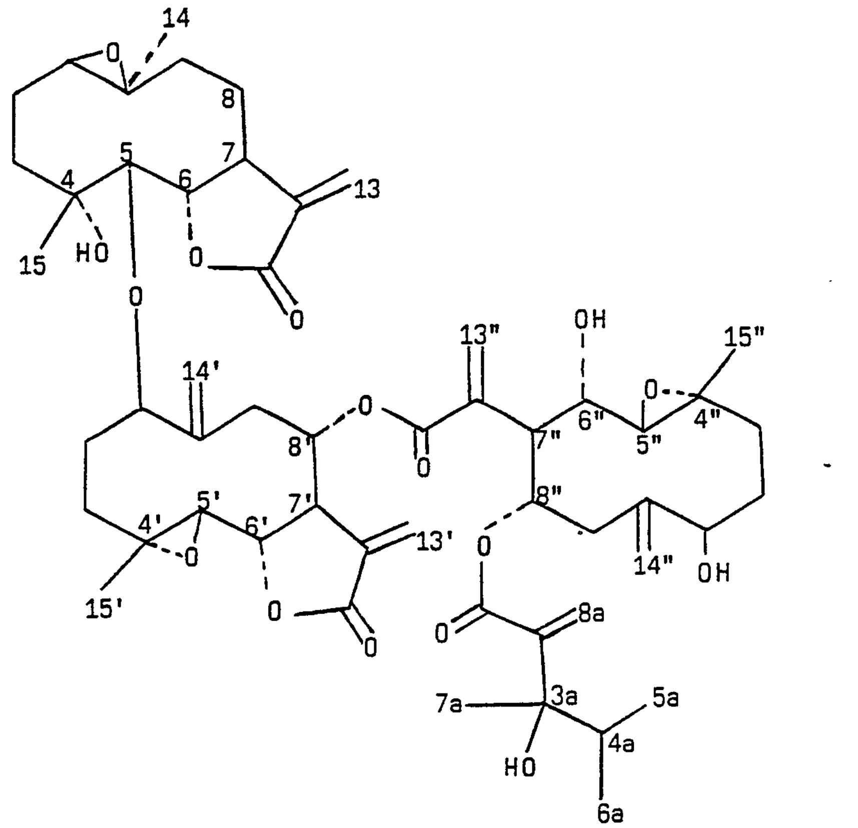

(5)

11. A method according to claim 10, which includes the additional step of hydrolysing the said compound of the formula (5) to obtain a compound of the formula

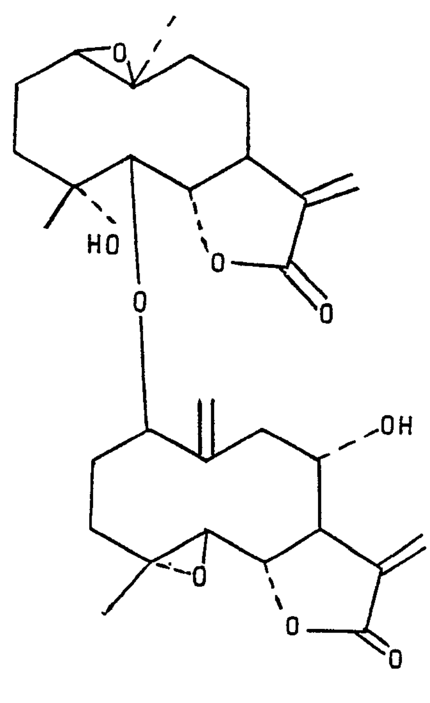

(6)

12. A method according to claim 8, wherein the or a said sesquiterpene lactone is a guaianolide which has the formula

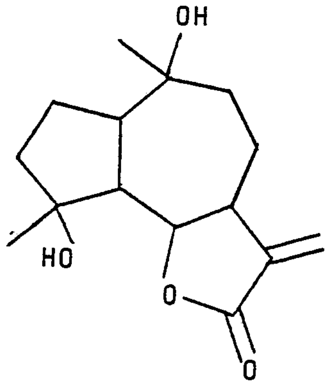

(3) '

or

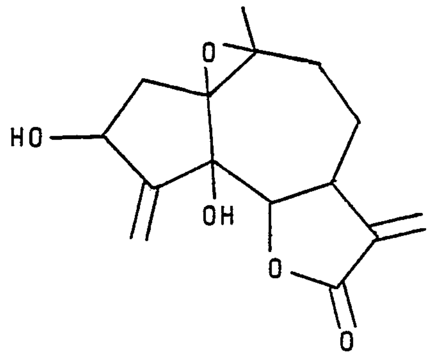

(4)

13. A method according to any one of claims 5 to 12, which comprises suspending particles of the dried said plant *Tanacetum parthenium* in the non-polar solvent and allowing the said solvent to extract the sesquiterpene lactone from the dried plant.

14. A method according to any one of claims 5 to 13, wherein the said further extraction is carried out by chromatography using the second non-polar solvent as at least a first eluate.

**Patentansprüche für die Vertragsstaaten: DE FR IT NL SE CH BE LU LI**

1. Spasmolytisch wirksame Zusammensetzung zur Verwendung bei der Behandlung von Migräne, Asthma, Bronchitis oder Arthritis, welche Zusammensetzung einen ein Sesquiterpen-Lacton enthaltenden Auszug bzw. Extrakt aus der Pflanze Tanacetum parthenium enthält, welcher Auszug in einem Öl gelöst ist.

2. Spasmolytisch wirksame Zusammensetzung nach Anspruch 1 für die Verwendung bei der Behandlung von Migräne.

3. Spasmolytisch wirksame Zusammensetzung nach Anspruch 1 enthaltend Teilchen der getrockneten Pflanze Tanacetum parthenium, die in dem genannten Öl suspendiert ist, wobei das Öl dazu dient, das Sesquiterpen-Lacton aus der getrockneten Pflanze zu extrahieren.

4. Spasmolytisch wirksame Zusammensetzung nach Anspruch 1, 2 oder 3, worin das Öl ein gesättiger oder ungesättigter, langkettiger Kohlenwasserstoff oder eine solche Fettsäure, ein pflanzliches oder tierisches Öl, oder ein polyoxyäthyliertes, rekonstituiertes Glycerid oder Esterderivat des pflanzlichen oder tierischen Öls ist.

5. Spasmolytisch wirksame Zusammensetzung nach Anspruch 4, worin das Öl Kokosnußöl, Sojabohnenöl oder Fischöl ist.

6. Spasmolytisch wirksame Zusammensetzung nach einem der vorhergehenden Ansprüche, die zusätzlich ein pharmazeutisch verträgliches Excipiens enthält.

7. Spasmolytisch wirksame Zusammensetzung nach einem der vorhergehenden Ansprüche, die für die perorale, rektale, parenterale Verabreichung oder Verabreichung durch Inhalation geeignet ist.

8. Spasmolytisch wirksame Zusammensetzung nach Anspruch 7, die die Form einer Tablette, Kapsel oder flüssigen Suspension aufweist.

9. Spasmolytisch wirksame Zusammensetzung nach Anspruch 8, die die Form einer eingekapselten Zubereitung aufweist, die in einem Öl suspendierte Teilchen der getrockneten Pflanze Tanacetum parthenium enthält, wobei das Öl dazu dient, das Sesquiterpen-Lacton aus der getrockneten Pflanze zu extrahieren.

10. Spasmolytisch wirksame Zusammensetzung nach einem der verhergehenden Ansprüche, die zusätzlich wenigstens ein bekanntes antiarthritisches Mittel enthält.

11. Spasmolytisch wirksame Zusammensetzung nach einem der Ansprüche 1 bis 9, die zusätzlich

wenigstens einen bekannten Bronchodilator, ein bekanntes Antihistamin oder ein bekanntes infektionshemmendes Mittel enthält.

12. Spasmolytisch wirksame Zusammensetzung nach einem der Ansprüche 1 bis 9, die weiters ein oder mehrere bekannte Antimigräne-Ingredienzien, Analgetika und/oder Antiemetika enthält.

13. Verwendung eines Sesquiterpen-Lactons zur Herstellung eines spasmolytisch wirksamen Arzneimittels für die Behandlung von Migräne, Asthma, Bronchitis oder Arthritis, welches Sesquiterpen-Lacton von der Pflanze Tanacetum parthenium nach einem Verfahren erhalten wird, das die Extraktion der Pflanze mit einem nichtpolaren organischen Lösungsmittel als wenigstens erstem Extraktionsmittel umfaßt.

14. Verwendung nach Anspruch 13, worin das nichtpolare Lösungsmittel Leichtpetroleum, Hexan oder Chloroform ist.

15. Verwendung nach Anspruch 14, worin das nichtpolare Lösungsmittel ein Öl ist.

16. Verwendung nach Anspruch 15, worin das Öl ein gesättigter oder ungesättigter langkettiger Kohlenwasserstoff oder eine solche Fettsäure, ein pflanzliches oder tierisches Öl oder ein polyoxyäthyliertes, rekonstituiertes Glycerid oder Ester des pflanzlichen oder tierischen Öls ist.

17. Verwendung nach Anspruch 16, worin das Öl Kokosnußöl, Sojabohnenöl oder Fischöl ist.

18. Verwendung nach einem der Ansprüche 13 bis 17, worin der Auszug bzw. Extrakt eine Mehrzahl von Sesquiterpen-Lactonen enthält.

19. Verwendung nach einem der Ansprüche 13 bis 18, worin das oder wenigstens ein Sesquiterpen-Lacton einen -Methylensubstituenten am Lactonring aufweist.

20. Verwendung nach Anspruch 19, worin das Sesquiterpen-Lacton ein Germacranolid ist, das ein Parthenolid der Formel

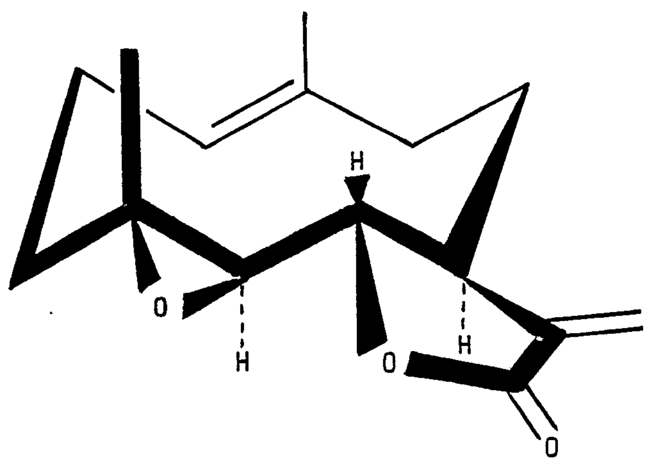

ist.

21. Verwendung nach Anspruch 19, worin das Sesquiterpen-Lacton ein Germacranolid ist, das wenigstens zwei Germacrakerne in seinem Molekül enthält.

22. Verwendung nach Anspruch 21, worin das Germacranolid die Formel

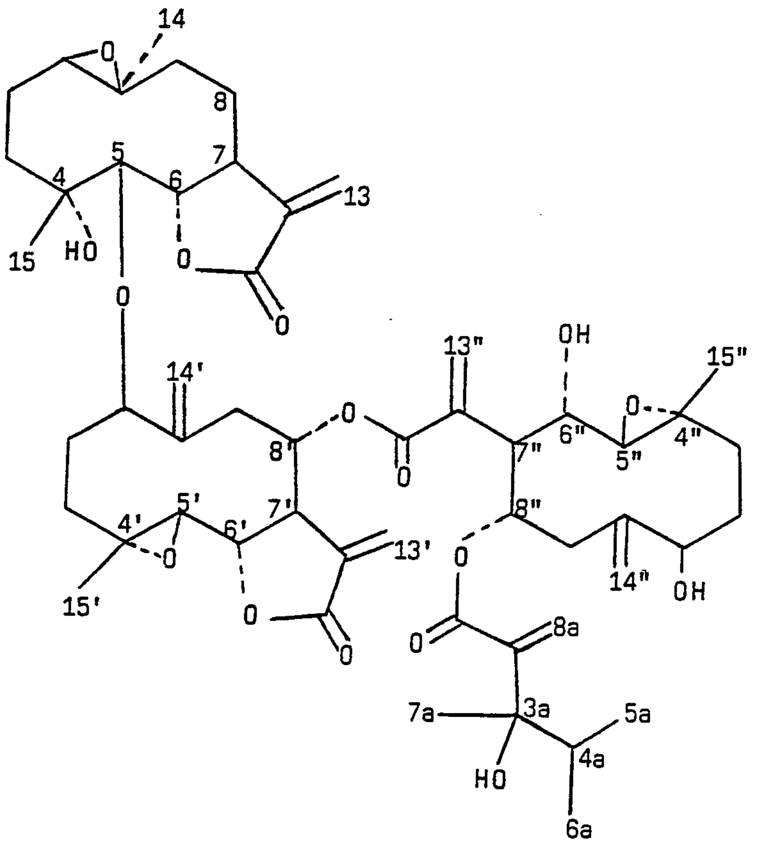

(5)

aufweist.

23. Verwendung nach Anspruch 13, worin das genannte Verfahren zur Extraktion der Pflanze weiters die Hydrolyse des Germacranolids (5) umfaßt, das in Anspruch 22 angeführt und definiert ist, um eine Verbindung der Formel

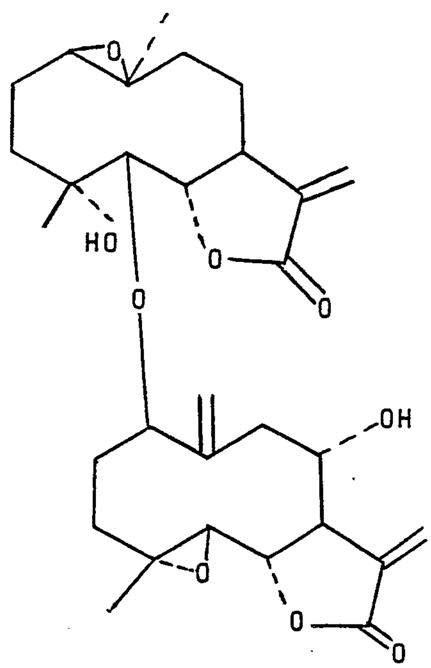

(6)

zu erhalten.

24. Verwendung nach Anspruch 19, worin das Sesquiterpen-Lacton ein Guaianolid ist, das die Formel

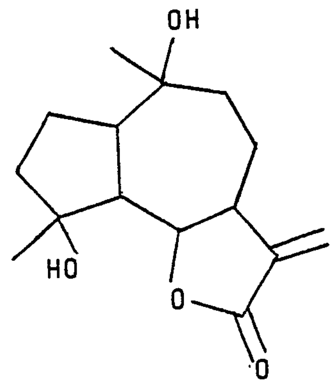

(3)'

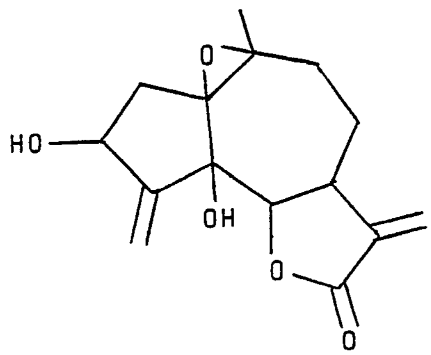

(4)

aufweist.

25. Verwendung nach einem der Ansprüche 13 bis 24 für die Behandlung von Migräne.

26. Sesquiterpen-Lacton, das aus der Pflanze Tanacetum parthenium erhalten wird und ein Germacranolid der Formel

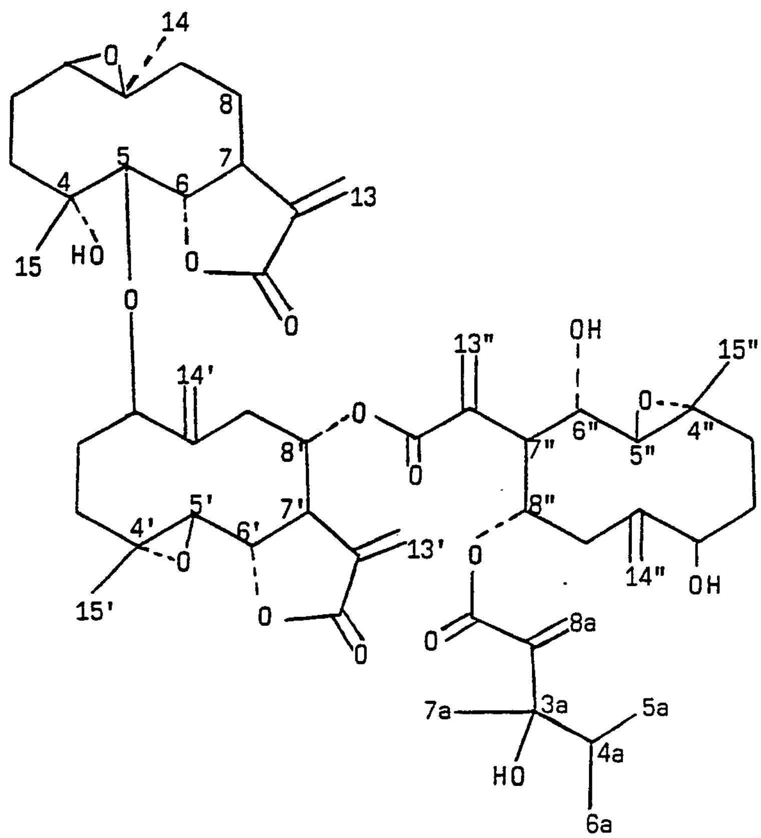

(5)

oder

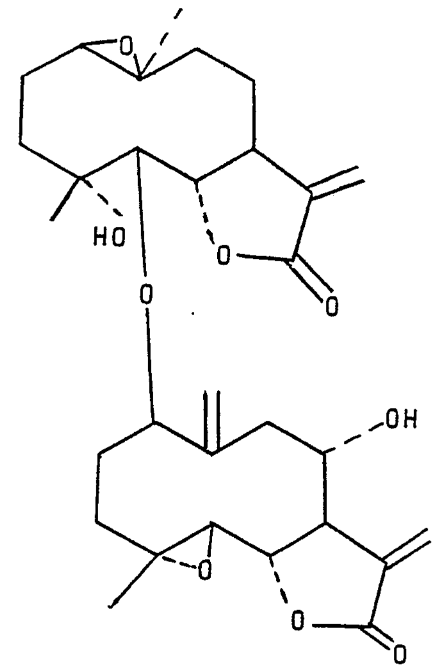

(6)

enthält.

27. Aus der Pflanze Tanacetum parthenium erhaltenes Sesquiterpen-Lacton, das ein Guaianolid der Formel

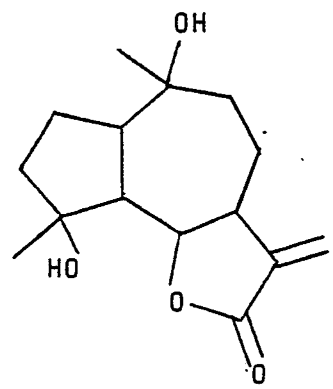

(3)'

oder

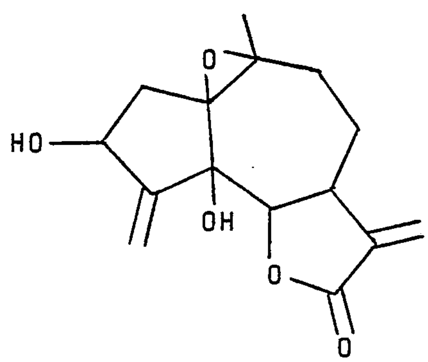

(4)

ist.

28. Sesquiterpen-Lacton nach Anspruch 26 oder 27, das im wesentlichen rein ist.

29. Spasmolytisch wirksame Zusammensetzung für die Verwendung bei der Behandlung von Migräne, Asthma, Bronchitis oder Arthritis, die ein Sesquiterpen-Lacton nach einem der Ansprüche 26 bis 28 und ein pharmazeutisch verträgliches Excipiens enthält.

30. Verfahren zur Herstellung eines Sesquiterpen-Lacton enthaltenden Auszugs bzw. Extraktes für pharmazeutische Zwecke, welches Verfahren die Behandlung von Tanacetum parthenium mit einem ersten nichtpolaren Lösungsmittel, Abdampfen des Lösungsmittels und Chromatographieren des entstandenen

rohen Auszugs bzw. Extraktes unter Verwendung eines zweiten nichtpolaren Lösungsmittels als wenigstens erstes Eluat umfaßt.

**Patentansprüche für den Vertragsstaat: AT**

1. Verfahren zur Herstellung einer spasmolytisch wirksamen Zusammensetzung für die Verwendung bei der Behandlung von Migräne, Asthma, Bronchitis oder Arthritis, welches Verfahren die Behandlung der Pflanze Tanacetum parthenium mit einem Öl umfaßt, in dem wenigstens eines der genannten Sesquiterpen-lactone löslich ist, und das genannte wenigstens eine Sesquiterpen-Lacton in dem Öl lösen gelassen wird.

2. Verfahren nach Anspruch 1, umfassend das Suspendieren von Teilchen der getrockneten Pflanze Tanacetum parthenium in dem genannten Öl.

3. Verfahren nach Anspruch 1 oder 2, worin das Öl ein gesättigter oder ungesättiger, langkettiger Kohlenwasserstoff oder eine solche Fettsäure, ein pflanzliches oder tierisches Öl, oder ein polyoxyäthyliertes, rekonstituiertes Glycerid oder Esterderivat des pflanzlichen oder tierischen Öls ist.

4. Verfahren nach Anspruch 3, worin das Öl Kokosnußöl, Sojabohnenöl oder Fischöl ist.

5. Verfahren zur Herstellung eines Sesquiterpen-Lacton enthaltenden Auszugs bzw. Extraktes für pharmazeutische Verwendung, welches Verfahren die Behandlung der Pflanze Tanacetum parthenium mit einem ersten nichtpolaren organischen Lösungsmittel, in dem wenigstens eines der genannten Sesquiterpen-Lactone löslich ist, Abdampfen des Lösungsmittels und Chromatographieren des rohen Auszugs bzw. Extraktes unter Verwendung eines zweiten nichtpolaren Lösungsmittels als wenigstens erstes Eluat umfaßt.

6. Verfahren nach Anspruch 5, worin der Auszug bzw. Extrakt eine Mehrzahl von Sesquiterpen-Lactonen enthält.

7. Verfahren nach Anspruch 5 oder 6, worin das nichtpolare Lösungsmittel Leichtpetroleum, Hexan, Chloroform oder ein Öl ist.

8. Verfahren nach Anspruch 5, 6 oder 7, worin das genannte oder eines der genannten Sesquiterpen-Lacton(e) einen -Methylensubstituenten am Lactonring aufweist.

9. Verfahren nach Anspruch 8, worin das genannte oder eines der genannten Sesquiterpen-Lacton(e) ein Germacranolid ist, das ein Parthenolid der Formel

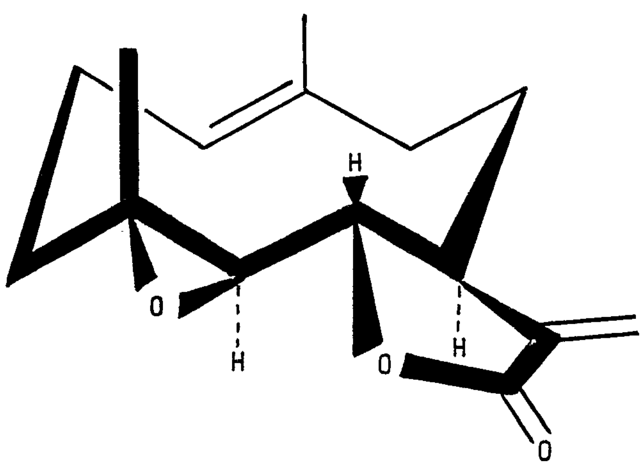

ist.

10. Verfahren nach Anspruch 8, worin das genannte oder eines der genannten Sesquiterpen-Lacton(e) ein Germacranolid ist, das die Formel

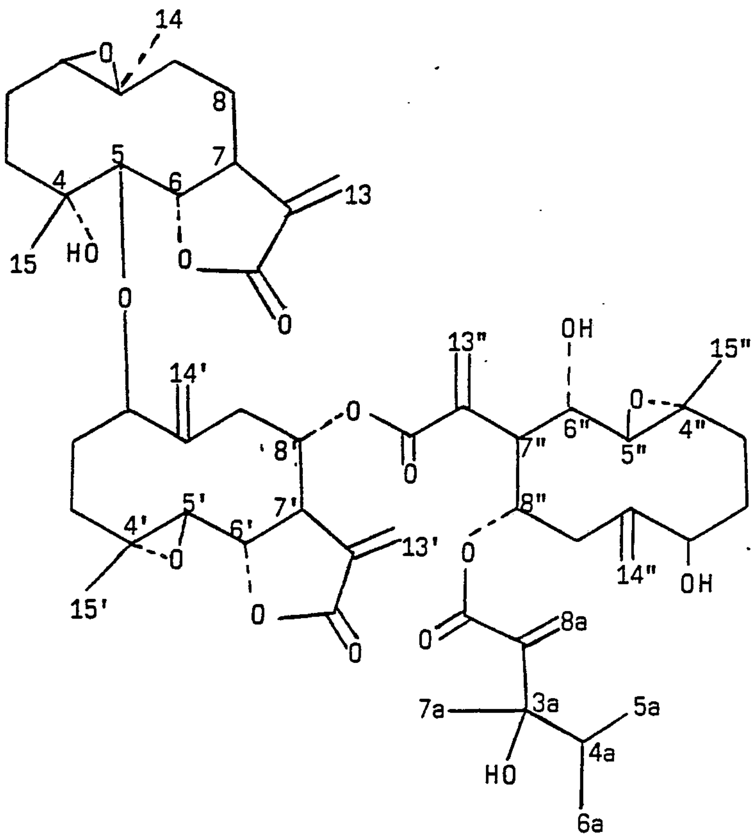

(5)

aufweist.

11. Verfahren nach Anspruch 10, das den zusätzlichen Schritt des Hydrolysierens der genannten Verbindung der Formel (5) umfaßt, um eine Verbindung der Formel

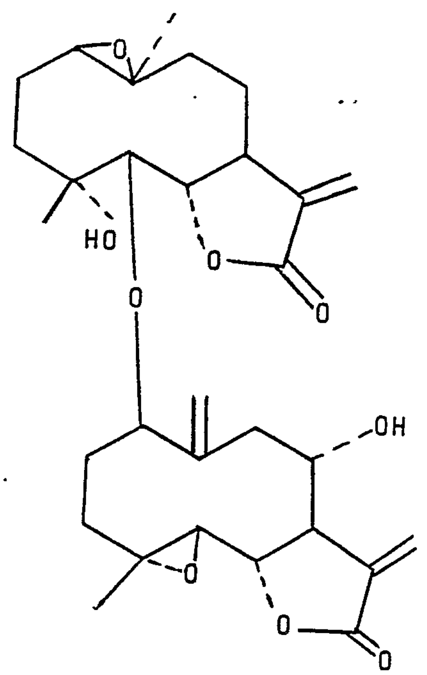

(6)

zu erhalten.

12. Verfahren nach Anspruch 8, worin das genannte oder eines der genannten Sesquiterpen-Lacton(e) ein Guaianolid ist, das die Formel

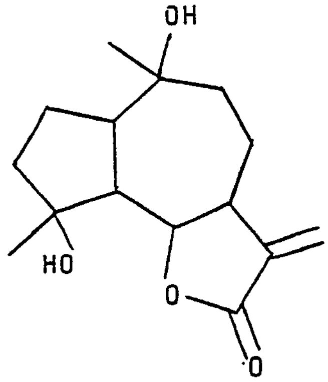

(3)'

oder

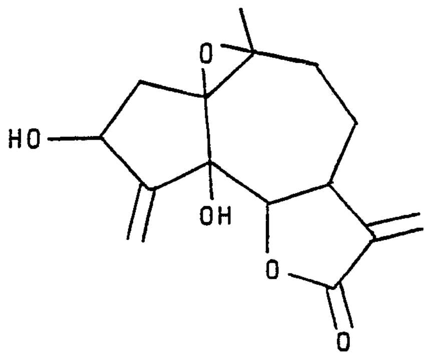

(4)

aufweist.

13. Verfahren nach einem der Ansprüche 5 bis 12, welches das Suspendieren von Teilchen der genannten getrockneten Pflanze Tanacetum parthenium in dem nichtpolaren Lösungsmittel und das Einwirkenlassen des genannten Lösungsmittels umfaßt, um das Sesqiterpen-Lacton aus der getrockneten Pflanze zu extrahieren.

14. Verfahren nach einem der Ansprüche 5 bis 13, worin die genannte weitere Extraktion durch Chromatographie unter Verwendung des zweiten nichtpolaren Lösungsmittels als wenigstens erstes Eluat durchgeführt wird.

**Revendications pour les Etats contractants: BE CH DE FR IT LI LU NL SE**

1. Composition spasmolytiquement active à utiliser dans le traitement d'une condition migraineuse, asthmatique, bronchique ou arthritique, laquelle composition comprend un extrait de la plante *Tanacetum parthenium* contenant des lactones sesquiterpéniques, lequel extrait est dissous dans une huile.

2. Composition spasmolytiquement active selon la revendication 1, à utiliser dans le traitement de la migraine.

3. Composition spasmolytiquement active selon la revendication 1, comprenant des particules de la plante séchée *Tanacetum parthenium* en suspension dans ladite huile, l'huile servant à extraire la lactone sesquiterpénique de la plante séchée.

4. Composition spasmolytiquement active selon la revendication 1, la revendication 2 ou la revendication 3, où l'huile est un hydrocarbure à chaîne longue saturé ou insaturé ou acide gras, une huile végétale ou animale ou un glycéride polyoxyéthylé reconstitué ou dérivé d'ester de l'huile végétale ou animale.

5. Composition spasmolytiquement active selon la revendication 4, où l'huile est de l'huile de noix de coco, de l'huile de soja ou de l'huile de poisson.

6. Composition spasmolytiquement active selon toute revendication précédente qui contient additionnellement un excipient acceptable en pharmacie.

7. Composition spasmolytiquement active selon l'une des revendications précédentes qui est appropriée pour une administration orale, rectale, parentérale ou par inhalation.

8. Composition spasmolytiquement active selon la revendication 7 qui est sous la forme d'un comprimé, d'une capsule ou d'une suspension liquide.

9. Composition spasmolytiquement active selon la revendication 8 qui a la forme d'une préparation encapsulée comprenant des particules de la plante séchée *Tanacetum parthenium* en suspension dans une huile, l'huile servant à extraire la lactone sesquiterpénique de la plante séchée.

10. Composition spasmolytiquement active selon toute revendication précédente, qui contient additionnellement au moins un agent anti-arthritique connu.

11. Composition spasmolytiquement active selon l'une des revendications 1 à 9, qui contient additionellement au moins un agent connu broncho-dilatateur, antihistaminique ou anti-infectieux.

12. Composition spasmolytiquement active selon l'une des revendications 1 à 9, qui contient de plus un ou plusieurs ingrédients connus anti-migraineux, analgésiques et/ou anti-émétiques.

13. Utilisation d'une lactone sesquiterpénique pour la fabrication d'un médicament spasmolytiquement actif pour le traitement d'une condition migraineuse, asthmatique, bronchique ou arthritique, laquelle lactone sesquiterpénique est dérivée de la plante *Tanacetum parthenium* par un procédé comprenant l'extraction de la plante par un solvant organiquement polaire en tant qu'au moins un premier agent d'extraction.

14. Utilisation selon la revendication 13, où le solvant non polaire est du pétrole léger, de l'hexane ou du chloroforme.

15. Utilisation selon la revendication 14, où le solvant non polaire est une huile.

16. Utilisation selon la revendication 15, où l'huile est un hydrocarbure à chaîne longue saturé ou insaturé ou acide gras, une huile animale ou végétale ou un glycéride polyoxyéthylé reconstitué ou ester de l'huile végétale ou animale.

17. Utilisation selon la revendication 16, où l'huile est l'huile de noix de coco, l'huile de soja ou une huile de poisson.

18. Utilisation selon l'une des revendications 13 à 17, où l'extrait contient un certain nombre de lactones sesquiterpéniques.

19. Utilisation selon l'une des revendications 13 à 18, où la ou au moins une lactone sesquiterpénique a un substituant α-méthylénique sur le noyau de lactone.

20. Utilisation selon la revendication 19, où la lactone sesquiterpènique est un germacranolide qui est un parthénolide de la formule

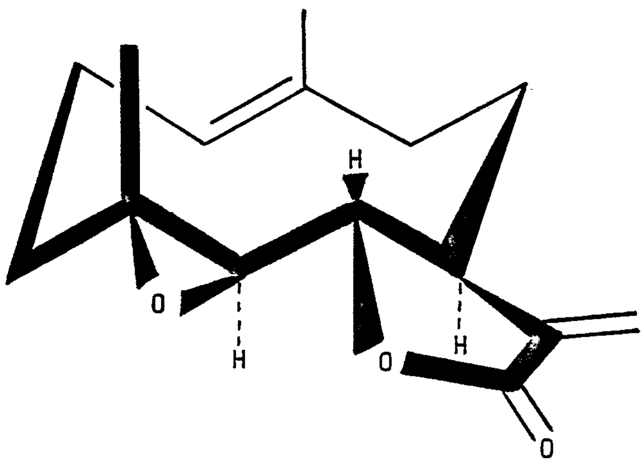

21. Utilisation selon la revendication 19, où la lactone sesquiterpénique est un germacranolide qui contient au moins deux noyaux de germacrane dans sa molécule.

22. Utilisation selon la revendication 21, où le germacranolide a pour formule

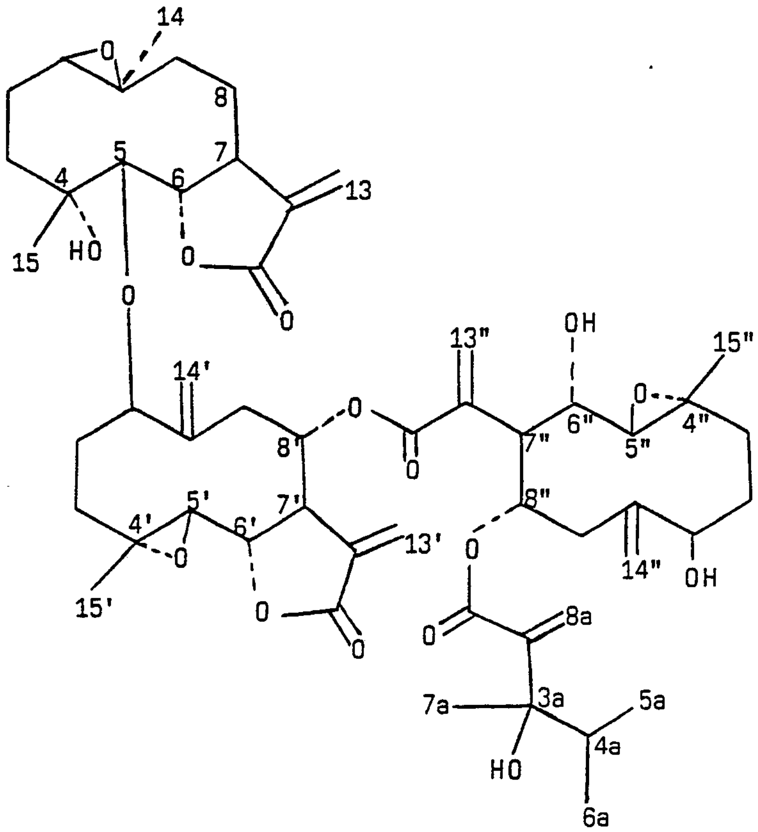

(5)

23. Utilisation selon la revendication 13, où ledit procédé comprenant l'extraction de la plante comprend de plus l'hydrolyse du germacranolide (5), donné et défini à la revendication 22, pour produire un composé ayant pour formule

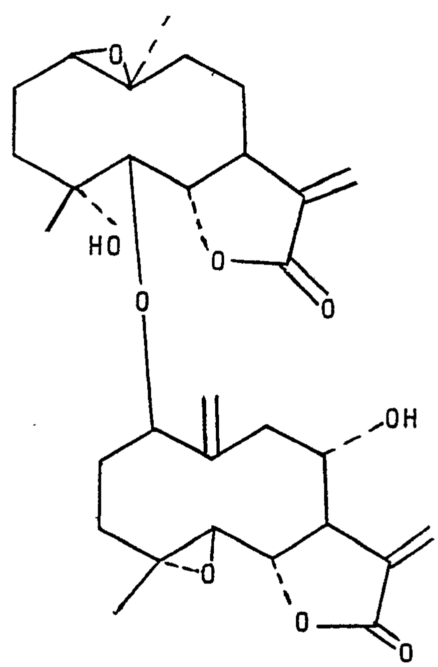

(6)

24. Utilisation selon la revendication 19, où la lactone sesquiterpénique est un guaianolide qui a pour formule

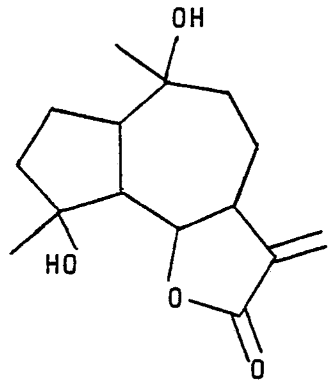

(3)'

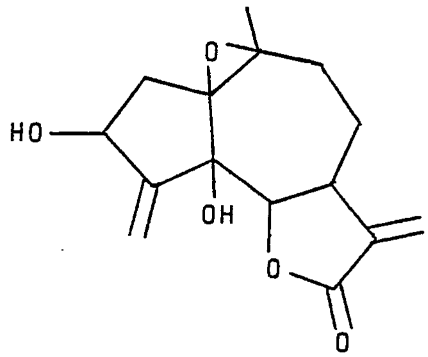

(4)

25. Utilisation selon l'une des revendications 13 à 24 pour le traitement de la migraine.

26. Lactone sesquiterpénique dérivée de la plante *Tanacetum parthenium* et comprenant un germacranolide ayant la formule

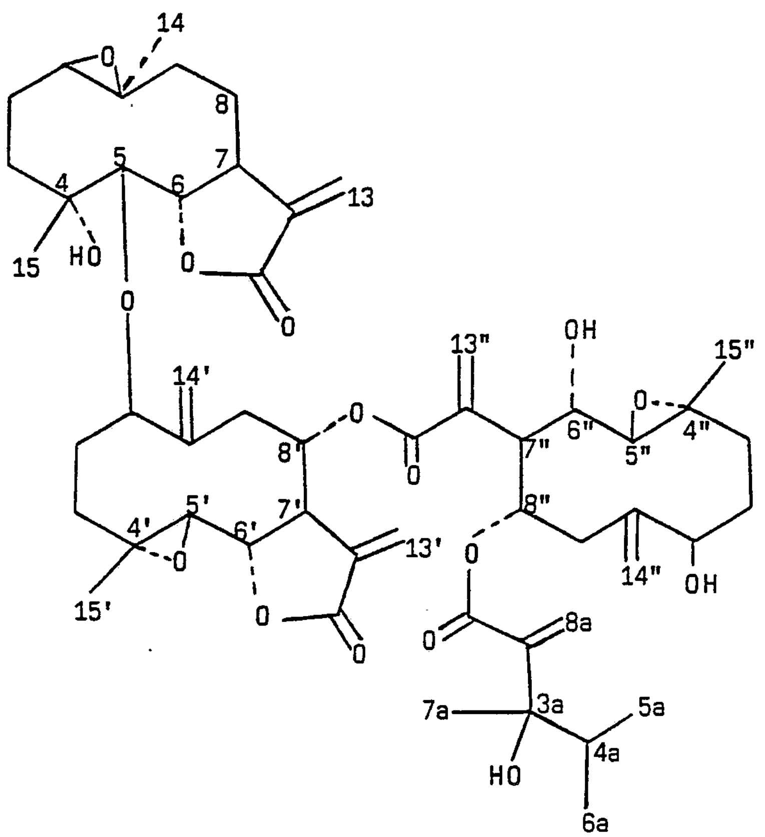

(5)

ou

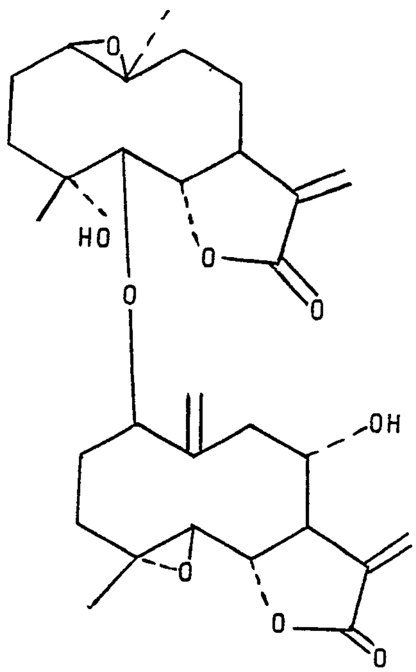

(6)

27. Lactone sesquiterpénique dérivée de la plante *Tanacetum parthenium* qui est un guaianolide ayant pour formule

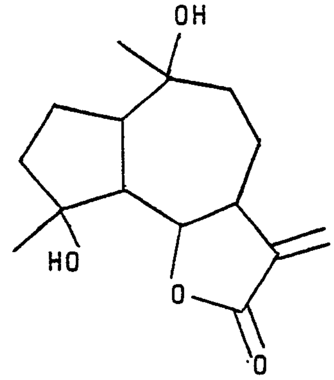

(3)'

ou

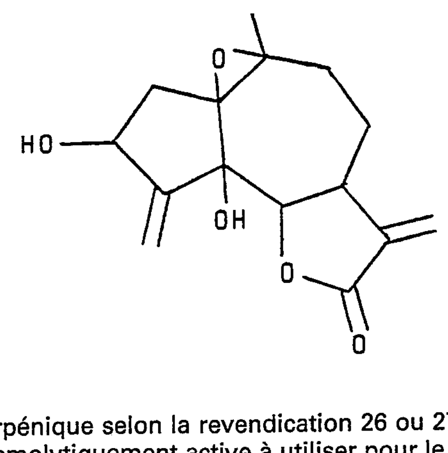

(4)

28. Lactone sesquiterpénique selon la revendication 26 ou 27 qui est essentiellement pure.

29. Composition spasmolytiquement active à utiliser pour le traitement d'une condition migraineuse, asthmatique, bronchique ou arthritique, comprenant une lactone sesquiterpénique selon l'une des revendications 26 à 28 et un excipient acceptable en pharmacie.

30. Méthode de préparation d'un extrait contenant une lactone sesquiterpénique pour un usage pharmaceutique, laquelle méthode comprend le traitement de *Tanacetum parthenium* avec un premier solvant non polaire, l'évaporation du solvant et la soumission de l'extrait brut résultant à une chromatographie en utilisant un second solvant non polaire comme au moins un premier éluat.

**Revendications pour l'Etat contractant: AT**

1. Méthode de préparation d'une composition spasmolytiquement active à utiliser dans le traitement d'une condition migraineuse, asthmatique, bronchique ou arthritique, laquelle méthode consiste à traiter la plante *Tanacetum parthenium* avec une huile dans laquelle au moins une lactone sesquiterpénique est soluble et à permettre à ladite au moins une lactone sesquiterpénique de se dissoudre dans l'huile.

2. Méthode selon la revendication 1, consistant à mettre des particules de la plante séchée *Tanacetum parthenium* en suspension dans ladite huile.

3. Méthode selon la revendication 1 ou la revendication 2, où l'huile est un hydrocarbure à chaîne longue saturé ou insaturé ou un acide gras, une huile animale ou végétale ou un glycéride polyoxyéthylé, reconstitué ou dérivé d'ester de l'huile végétale ou animale.

4. Méthode selon la revendication 2, où l'huile est l'huile de noix de coco, l'huile de soja ou l'huile de poisson.

5. Méthode de préparation d'un extrait contenant une lactone sesquiterpénique pour un usage pharmaceutique, laquelle méthode consiste à traiter la plante *Tanacetum parthenium* avec un premier solvant organique non polaire dans lequel au moins une lactone sesquiterpénique est soluble, à évaporer le solvant et à soumettre l'extrait brut à une chromatographie en utilisant un second solvant non polaire en tant qu'au moins un premier éluat.

6. Méthode selon la revendication 5, où l'extrait contient un certain nombre de lactones sesquiterpéniques.

7. Méthode selon la revendication 5 ou la revendication 6, où le solvant non polaire est du pétrole léger, de l'hexane, du chloroforme ou une huile.

8. Méthode selon la revendication 5, la revendication 6 ou la revendication 7, où la ou une lactone sesquiterpénique a un substituant α-méthylénique sur le noyau de lactone.

9. Méthode selon la revendication 8, où la ou une lactone sesquiterpénique est un germacranolide qui est un parthénolide de la formule

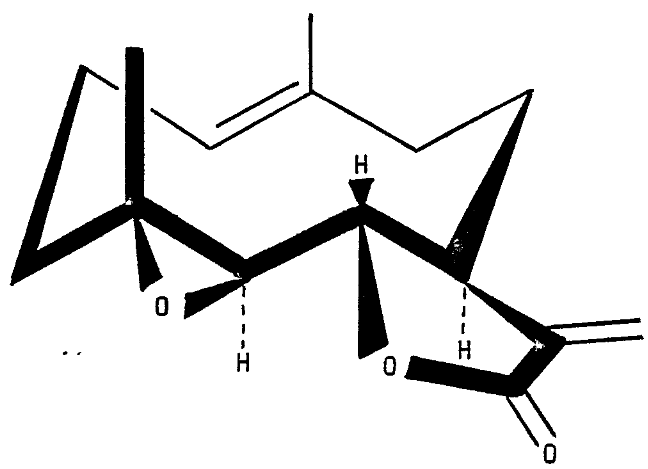

10. Méthode selon la revendication 8, où la ou une lactone sesquiterpénique comprend un germacranolide ayant la formule

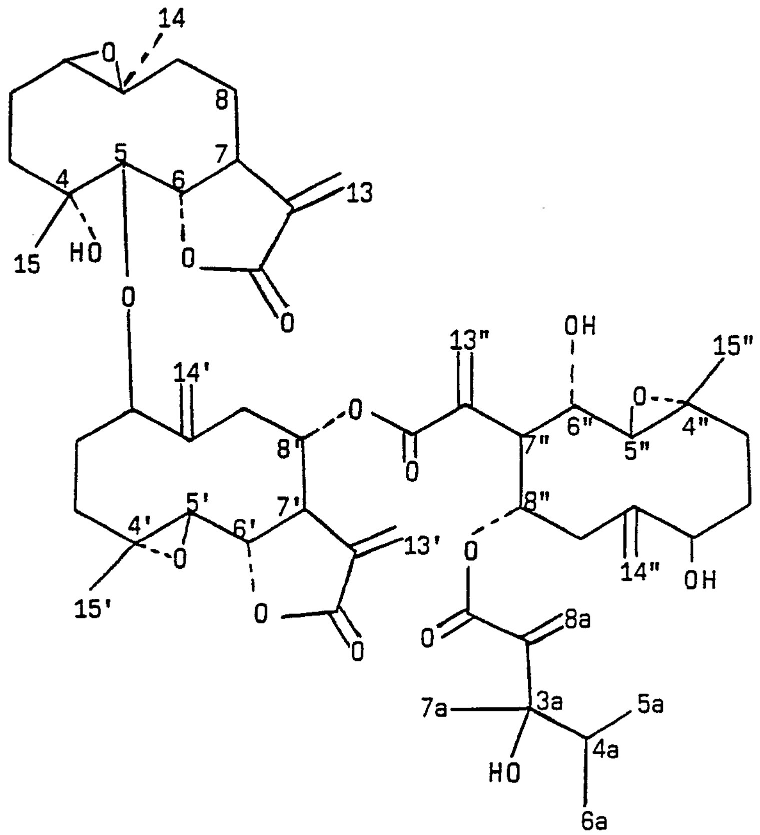

(5)

11. Méthode selon la revendication 10, qui comprend l'étape additionnelle d'hydrolyser ledit composé de la formule (5) pour obtenir un composé de formule

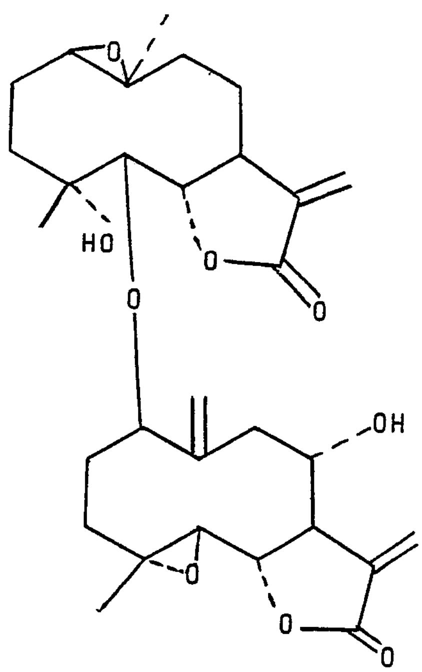

(6)

12. Méthode selon la revendication 8, où la ou une lactone sesquiterpénique est un guaianolide qui a pour formule

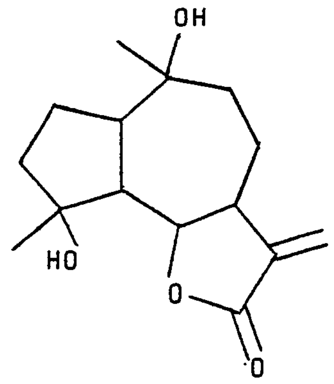

(3)'

ou

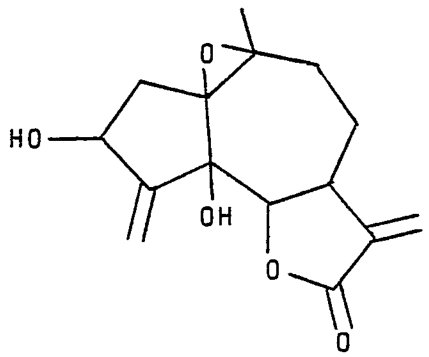

(4)

13. Méthode selon l'une des revendications 5 à 12, qui consiste à mettre les particules de ladite plante séchée *Tanacetum parthenium* en suspension dans le solvant non polaire et à permettre audit solvant d'extraire la lactone sesquiterpénique de la plante séchée.

14. Méthode selon l'une des revendications 5 à 13, où ladite autre extraction est effectuée par chromatographie en utilisant le second solvant non polaire en tant qu'au moins un premier éluat.

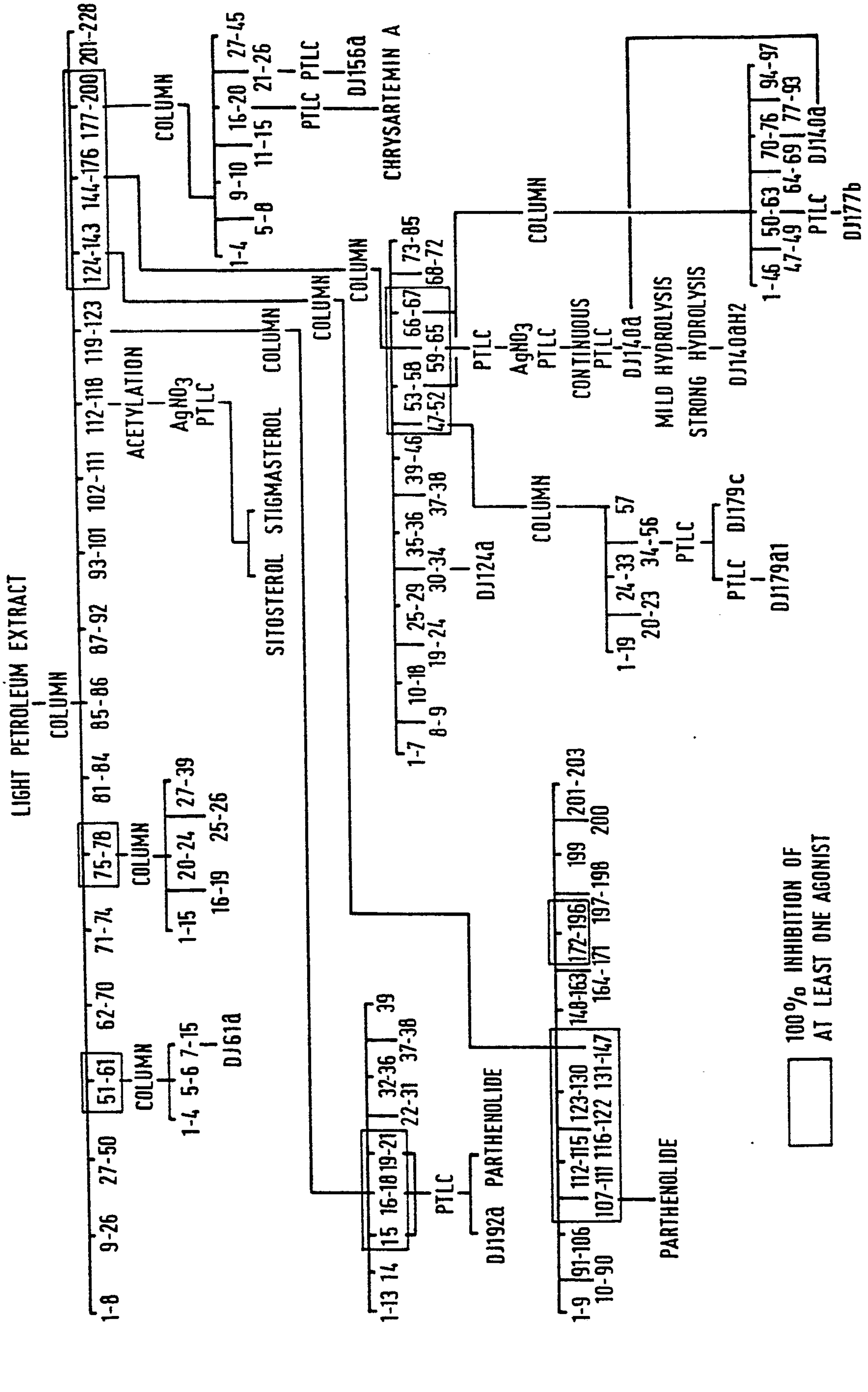
LIGHT PETROLEUM EXTRACT
COLUMN
1-8
9-26
27-50
51-61
62-70
71-74
75-78
81-84
85-86
87-92
93-101
102-111
112-118
119-123
124-143
144-176
177-200
201-228
COLUMN
1-4 5-6 7-15
DJ61a
COLUMN
1-15 16-19 20-24 25-26 27-39
ACETYLATION
AgNO3
PTLC
SITOSTEROL
STIGMASTEROL
COLUMN
1-13 14 15 16-18 19-21 22-31 32-36 37-38 39
PTLC
PARTHENOLIDE
DJ192a
COLUMN
1-9 10-90 91-106 107-111 112-115 116-122 123-130 131-147 148-163 164-171 172-196 197-198 199 200 201-203
PARTHENOLIDE
COLUMN
1-7 8-9 10-18 19-24 25-29 30-34 35-36 37-38 39-46 47-52 53-58 59-65 66-67 68-72 73-85
DJ124a
PTLC
AgNO3
PTLC
CONTINUOUS
PTLC
DJ140a
MILD HYDROLYSIS
STRONG HYDROLYSIS
DJ140aH2
COLUMN
1-19 20-23 24-33 34-56 57
PTLC
PTLC DJ179c
DJ179a1
COLUMN
1-46 47-49 50-63 64-69 70-76 77-93 94-97
PTLC
DJ177b
DJ140a
COLUMN
1-4 5-8 9-10 11-15 16-20 21-26 27-45
PTLC PTLC PTLC
DJ156a
CHRYSARTEMIN A
100% INHIBITION OF
AT LEAST ONE AGONIST